(19) Europäisches Patentamt
European Patent Office
Office européen des brevets

(11) Veröffentlichungsnummer : **0 045 842**
**B1**

(12) # EUROPÄISCHE PATENTSCHRIFT

(45) Veröffentlichungstag der Patentschrift : **18.01.84**

(21) Anmeldenummer : **81104982.4**

(22) Anmeldetag : **26.06.81**

(51) Int. Cl.³ : **C 07 D307/935**, A 61 K 31/34// C07C177/00

(54) **Heterocyclische Verbindungen, diese enthaltende Arzneimittel und Verfahren zur Herstellung dieser heterocyclischen Verbindungen und Arzneimittel.**

(30) Priorität : **08.08.80 DE 3029984**

(43) Veröffentlichungstag der Anmeldung : **17.02.82 Patentblatt 82/07**

(45) Bekanntmachung des Hinweises auf die Patenterteilung : **18.01.84 Patentblatt 84/03**

(84) Benannte Vertragsstaaten :
**AT BE CH DE FR GB IT LI LU NL SE**

(56) Entgegenhaltungen :
**EP-A- 0 009 869**
**DE-A- 2 702 553**
**Die Akte enthält technische Angaben, die nach dem Eingang der Anmeldung eingereicht wurden und die nicht in dieser Patentschrift enthalten sind.**

(73) Patentinhaber : **Grünenthal GmbH**
**Patentabteilung Steinfeldstrasse 2**
**D-5190 Stolberg (DE)**

(72) Erfinder : **Seipp, Ulrich, Dr.**
**Ludwigsallee 81**
**D-5100 Aachen (DE)**
Erfinder : **Vollenberg, Werner, Dr.**
**Eupener Strasse 26a**
**D-5190 Stolberg (DE)**
Erfinder : **Michel, Gundrun, Dr., geb. Lambert**
**von-Coels-Strasse 98**
**D-5100 Aachen (DE)**
Erfinder : **Müller, Bernd, Dr.**
**Sonnenscheinstrasse 30**
**D-5100 Aachen (DE)**

**EP 0 045 842 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Heterocyclische Verbindungen, diese enthaltende Arzneimittel und Verfahren zur Herstellung dieser heterocyclischen Verbindungen und Arzneimittel

Die vorliegende Erfindung betrifft heterocyclische Verbindungen der Formel

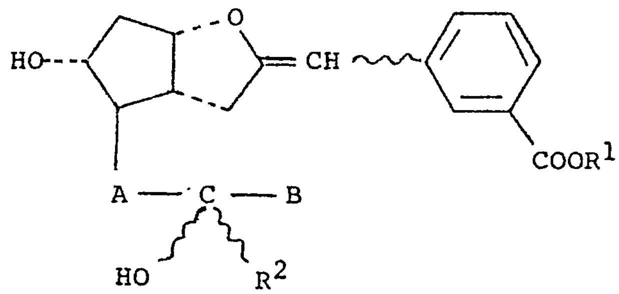

(I)

deren Grundgerüst auch zum Beispiel im natürlich vorkommenden Prostacyclin enthalten ist.

$R^1$ bedeutet ein Wasserstoffatom, ein pharmazeutisch verträgliches Kation oder einen geradkettigen oder verzweigten Alkylrest mit 1-6 Kohlenstoffatomen. Vorzugsweise steht jedoch $R^1$ für einen Methyl- oder Äthylrest oder, wenn diese Gruppe ein Kation bedeutet, für ein Natrium- oder Kaliumion. Andere geeignete Kationen sind aus der Prostaglandin- bzw. Prostacyclinchemie bekannt. Beispielsweise kommen auch Calcium- oder Magnesiumionen, Ammoniumionen und Aminionen, wie die von Mono-, Di- oder Tri-methylamin, -äthylamin, -äthanolamin, Tris-hydroxymethyl-amin usw. in Betracht, und ferner können für die Salzbildung auch basische Aminosäuren wie Arginin oder Lysin herangezogen werden.

$R^2$ bedeutet Wasserstoff oder einen Methylrest, wobei das Kohlenstoffatom (15), an das unter anderem der Rest $R^2$ gebunden ist, in den Verbindungen der Formel I in der RS- oder in der S-Konfiguration vorliegen kann. Bevorzugt sind die 15S-Formen der Verbindungen der Formel I.

A bedeutet eine der Gruppen $—CH_2—CH_2—$, (trans)-CH = CH— oder —C ≡ C— wobei die beiden letzteren dieser Gruppen, insbesondere aber die trans-CH = CH-Gruppierung speziell in den 15S-Formen der Verbindungen der Formel I bevorzugt sind.

B stellt einen Alkylrest mit 5-9 Kohlenstoffatomen, der die Struktur

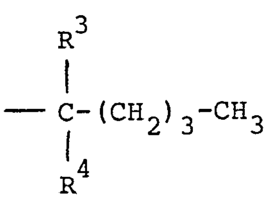

hat, in der $R^3$ und $R^4$ gleich oder verschieden sind und für Wasserstoff, Methyl oder Äthyl stehen oder einen Cyclohexylrest, der in der 4'-Stellung durch einen Methyl- oder Äthylrest substituiert sein kann, dar. Bevorzugte Bedeutungen von B sind der Cyclohexylrest oder der Alkylrest der vorstehend angegebenen Struktur, in dem $R^3$ und $R^4$ jeweils für Wasserstoff oder jeweils für Methyl stehen bzw. worin $R^3$ für Wasserstoff und $R^4$ für Äthyl steht.

In der Formel I kann der Phenylrest in Bezug auf die Doppelbindung E-, EZ- oder vorzugsweise Z-Konfiguration aufweisen.

Hat A die insbesondere bevorzugte trans-CH = CH-Struktur, so entsprechen die erfindungsgemäßen Verbindungen der folgenden Formel

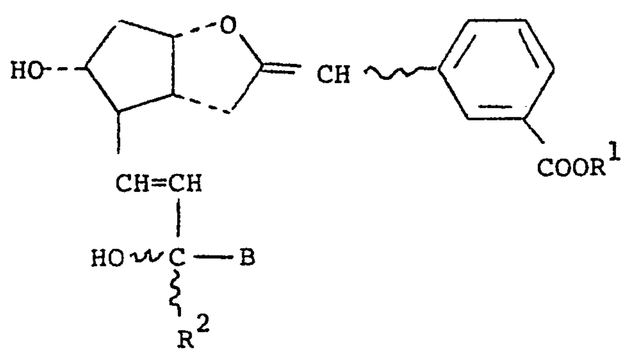

worin $R^1$, $R^2$ und B die gleiche Bedeutung wie in Formel I haben. Entsprechend kommen den besonders bevorzugten Verbindungen, in denen A eine trans-CH = CH-Gruppe und B der oben bezeichnete Alkylrest oder Cycloalkylrest ist, folgende Strukturen zu

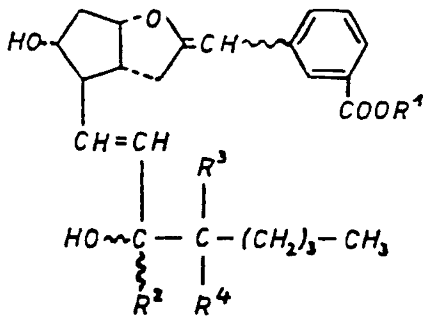

bzw.

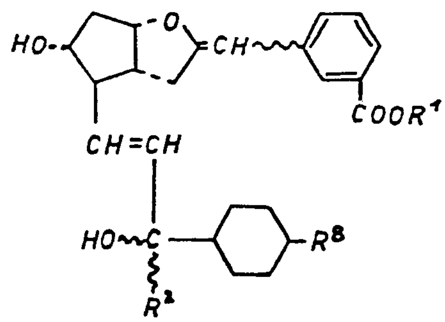

worin $R^1$ bis $R^4$ bzw. $R^1$ und $R^2$ die gleiche Bedeutung wie oben haben und worin $R^8$ für Wasserstoff, Methyl oder Äthyl steht. ·

Zur Nomenklatur der Verbindungen der Formel I sei auf die Arbeiten von Nelson in J. Med. Chem. *17,* 911 (1974) und Johnson et al. in Prostaglandins *15,* 737 (1978) Bezug genommen. In Anlehnung an die dort gemachten Angaben werden die Kohlenstoffatome im Gerüst der Verbindungen der Formel I wie folgt numeriert:

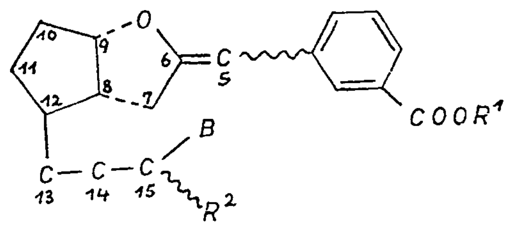

worin entsprechend der Definition von A zwischen den Kohlenstoffatomen 13 und 14 auch eine Doppel- oder Dreifachbindung liegen kann und worin $R^1$, $R^2$ und B die gleiche Bedeutung wie in Formel I haben, wobei hier auf die Bezifferung der Kohlenstoffatome in diesen Resten nicht näher eingegangen zu werden braucht. Es sei lediglich erwähnt, daß, wenn B für einen Cyclohexylrest steht, dessen Kohlenstoffatome als 1', 2' usw. bezeichnet werden sollen (wie auch aus der Definition von B ersichtlich).

Z. B. aus der EP-A 9869 und der DE-A 27 02 553 sind bereits viele Derivate bzw. Abkömmlinge des Prostacyclins bekannt, die u. a. in ihrem Molekül auch Phenylreste enthalten können. Diese sind aber entweder in Form gegebenenfalls substituierter Phenolreste esterartig mit der Carboxylgruppe des Prostacyclins verknüpft oder die Phenyl- bzw. Arylreste sind an eines der Kohlenstoffatome 15 oder 16 etc. des Prostacyclins gebunden. Die DE-A 27 02 553 betrifft ferner solche Abkömmlinge des Prostacyclins, in denen die Carboxylgruppe völlig durch einen Tetrazolyl-5-rest oder z. B. durch eine Dialkylaminomethylgruppe ersetzt ist. Da bekannt ist, daß der Ersatz einer Carboxylgruppe durch einen Tetrazolylrest in einer pharmakologisch wirksamen Substanz zu einem zumindest qualitativ gleichwirkenden Produkt führt und ferner bekannt ist, daß der Ersatz der Carboxylgruppe in Prostaglandinen durch eine Dimethylaminogruppe zu Prostaglandin-Antagonisten führt (J. Pharmacol. Exp. Ther. *206* (1978) 139), ließen sich aufgrund des Standes der Technik keinerlei Vorhersagen machen, wie sich der erstmalige Ersatz der aliphatischen Kette der Kohlenstoffatome 2, 3 und 4 im Prostacyclinmolekül durch einen Phenylenring in pharmakologischer Hinsicht sowie in Bezug auf Zugänglichkeit und Stabilität der resultierenden Produkte auswirken würde.

Es ist daher nicht nur überraschend, daß die Verbindungen der Formel I auf die Blutplättchenaggregation in vitro und in vivo sowie auf den Blutdruck prostacyclinartig wirken, sondern insbesondere auch, daß sie sich gegenüber dem Prostacyclin z. B. durch eine erheblich gesteigerte Stabilität auszeichnen. Im Vergleich zu dem als chemisch stabile Referenzsubstanz herangezogenen 5,6-Dihydroprostacyclin erreicht z. B. das Produkt des Beispiels 3b ein Vielfaches der Wirkung der Vergleichssubstanz. Besonders überraschend ist bei den erfindungsgemäßen Substanzen das Verhältnis von thrombozytenaggregationshemmender zu blutdrucksenkender Wirksamkeit: im Gegensatz zu 5,6-Dihydroprostacyclin, bei dem beide Effekte im gleichen Dosisbereich auftreten, werden bei den Verbindungen der Formel I blutdrucksenkende Effekte erst in wesentlich höheren Dosen beobachtet als die aggregationshemmende Wirkung, wie sich aus den nachfolgenden Tabellen (1a-1d) ergibt, in denen für einige Substanzen der Formel I die experimentell (bei den in-vivo-Versuchen an Gruppen von jeweils 4 bis 6 Tieren) gefundenen Werte für die relative Wirksamkeit im Vergleich zu 5,6-Dihydroprostacyclin aufgeführt sind:

Tabelle 1a

Relative Wirksamkeit auf die durch Arachidonsäure induzierte Aggregation von Humanthrombozyten

in vitro (die $IC_{50}$, d. h. die Konzentration, die in 50 % der Fälle unter den Versuchsbedingungen eine Thrombozyten-Aggregation verhindert, beträgt für 5,6-Dihydroprostacyclin 0,18 μ Mol/l) :

| Prüf-substanz | 5,6-Dihydro-prosta-cyclin | Produkt des Beispiels | | | | | |
|---|---|---|---|---|---|---|---|
| | | 1e)ii) | 1e)i) | 2a | 2b | 3b | 4b |
| relative Wirk-samkeit | 1,0 | 0,25 | 0,86 | 1,2 | 1,2 | 18,0 | 0,31 |

Tabelle 1b

Relative Wirksamkeit auf die ADP-induzierte Thrombozytopenie in vivo an der narkotisierten Ratte (Urethannarkose ; Verabreichung der Prüfsubstanzen intravenös ; die $ED_{50}$ für 5,6-Dihydroprostacyclin beträgt unter diesen Versuchsbedingungen 0,011 4 mg/kg) :

| Prüfsubstanz | 5,6-Dihydro-prosta-cyclin | Produkt des Beispiels | | | |
|---|---|---|---|---|---|
| | | 1e)ii | 1e)i) | 3b | 4b |
| relative Wirk-samkeit | 1,0 | 0,97 | 1,3 | 5,0 | 0,2 |

Dabei ist bemerkenswert, daß insbesondere das Produkt des Beispiels 3b sowohl in vitro (Tabelle 1a) als auch in vivo (Tabelle 1b) vielfach wirksamer als 5,6-Dihydroprostacyclin ist.

Tabelle 1c

Relative blutdrucksenkende Wirkung an wachen, spontan hypertonen Ratten (Messung über Dauerkatheter ; intravenöse Applikation der Prüfsubstanzen ; die $ED_{20}$ für 5,6-Dihydroprostacyclin beträgt unter diesen Versuchsbedingungen 0,005 mg/kg) :

| Prüfsubstanz | 5,6-Dihydro-prosta-cyclin | Produkt des Beispiels | | | |
|---|---|---|---|---|---|
| | | 1e)ii) | 1e)i) | 3b | 4b |
| relative Wirk-samkeit | 1,0 | 0,014 | 0,1 | 0,25 | 0,012 |

Tabelle 1d

Selektivitätsindex (aggregationshemmende Wirksamkeit/blutdrucksenkende Wirksamkeit) im Vergleich zu 5,6-Dihydroprostacyclin (aus den Werten der Tabellen 1b und 1c errechnet)

| Prüfsubstanz | 5,6-Dihydro-prosta-cyclin | Produkt des Beispiels | | | |
|---|---|---|---|---|---|
| | | 1e)ii) | 1e)i) | 3b | 4b |
| Selektivitäts-index | 1,0 | 69,3 | 13,0 | 20,0 | 16,7 |

Aus diesen Befunden ergibt sich, daß die Verbindungen der Formel I sowohl bei Krankheitszuständen verwendbar sind, bei denen eine Aggregationshemmung ohne begleitende Blutdrucksenkung erwünscht ist (z. B. Hyperaggregabilität bei koronarer Herzkrankheit) als auch in höheren Dosen bei Krankheitszuständen, bei denen eine begleitende gefäßerweiternde Wirkung zweckmäßig ist (z. B. periphere arterielle Verschlußkrankheiten).

Natürliches Prostacyclin hat ebenso wie das chemisch stabile 5,6-Dihydroprostacyclin in vivo nur eine kurze aggregationshemmende und blutdrucksenkende Wirkung. Diese Verbindungen können deshalb therapeutisch nur in Form einer Dauerinfusion appliziert werden. Abgesehen von der spontan hydrolytischen Inaktivierung des natürlichen Prostacyclins sind die Inaktivierungsmechanismen, die zu der nur kurzen Wirkdauer auch des chemisch stabilen 5,6-Dihydroprostacyclins in vivo führen, zur Zeit nur teilweise aufgeklärt.

Überraschenderweise besitzen die Verbindungen der Formel I eine wesentlich länger anhaltende Wirkung als das 5,6-Dihydroprostacyclin, d. h., daß sie, wie sich aus den nachfolgenden Tabellen 2a und 2b ergibt, auch für die Erzielung langanhaltender aggregationshemmender und blutdrucksenkender Wirkungen nach Gabe von Einzeldosen geeignet sind.

Tabelle 2a

Wirkungsdauer der Thrombozyten-aggregationshemmung in vivo (gemessen am Modell der ADP-induzierten Thrombozytopenie an der narkotisierten Ratte bei intravenöser Verabreichung der Prüfsubstanzen):

| Prüfsubstanz | Dosis (mg/kg i.v.) | Maximaleffekt % Aggregationshemmung | Wirkung bez. auf Maximaleffekt | |
|---|---|---|---|---|
| | | | 10 min nach Applikation | 30 min nach Applikation |
| 5,6-Dihydroprostcyclin | 0,1 | 62,4 = 100 % | 33,8 % | 0 % |
| Beispiel 1e)i) | 1,0 | 72,7 = 100 % | 69,3 % | 41,3 % |
| Beispiel 3b | 0,1 | 72,5 = 100 % | 62,6 % | 66,3 % |
| Beispiel 4b | 1,0 | 66,8 = 100 % | 45,4 % | 24,5 % |

Tabelle 2b

Blutdrucksenkende Wirkung an wachen, spontan hypertonen Ratten

| Prüfsubstanz | Dosis (mg/kg i.v.) | Maximaleffekt % Blutdrucksenkung | Wirkung bez. auf Maximaleffekt | |
|---|---|---|---|---|
| | | | 10 min nach Applikation | 30 min nach Applikation |
| 5,6-Dihydroprostacyclin | 0,0215 | 28 = 100 % | 7,1 % | 0 % |
| Beispiel 3b | 0,1 | 23 = 100 % | 87,0 % | 39,1 % |
| Beispiel 4b | 1,0 | 22 = 100 % | 90,9 % | 68,2 % |

Prostaglandine wie $PGE_2$ und $PGF_{2\alpha}$ wirken auf die glatte Muskulatur von Darm und Uterus Kontrahierend und können so in vivo Diarrhoen und Aborte auslösen. Bei Prostacyclinanalogen, die zur

Hemmung der Thrombozytenaggregation und Blutdrucksenkung benutzt werden, müssen solche Effekte als unerwünscht gelten.

Die neuen Substanzen der allgemeinen Formel I besitzen, wie sich aus nachfolgenden Tabellen 3a und 3b ergibt, im Gegensatz zum 5,6-Dihydroprostacyclin nur äußerst geringe spasmogene Eigenschaften, so daß bei therapeutischer Anwendung in antiaggregatorischen und blutdrucksenkenden Dosen nicht mit entsprechenden Nebenwirkungen zu rechnen ist.

Tabelle 3

Relative spasmogene Wirkung im Vergleich zu $PGF_{2\alpha}$ und 5,6-Dihydroprostacyclin

a) isolierter Rattenuterus

| Substanz | relative spasmogene Wirkung |
|---|---|
| $PGF_{2\alpha}$ | 100 % ($EC_{50}$ 50,1 µM) |
| 5,6-Dihydroprostacyclin | 26,1 % |
| Aus Beispiel 1e)i) | 0,58 % |

b) isoliertes Rattencolon

| Substanz | relative spasmogene Wirkung |
|---|---|
| $PGF_{2\alpha}$ | 100 % ($EC_{50}$ 50,14 µM) |
| 5,6-Dihydroprostacyclin | 23,8 % |
| Aus Beispiel 1e)i) | 0,002 % |

Entsprechende Befunde wurden auch mit anderen Substanzen der Formel I erhalten.

Da die erfindungsgemäßen Substanzen der Formel I sich nicht nur durch überraschende wertvolle biologische Eigenschaften, sondern auch durch gute chemische Stabilität auszeichnen, eignen sie sich sowohl zur parenteralen als auch zur oralen Verabreichung zur Hemmung der Thrombozytenaggregation am Menschen, und zwar zur Vorbeugung und Behandlung von Krankheitszuständen, bei denen die Thrombozytenaggregation und/oder eine Hyperaggregabilität pathogenetisch von Bedeutung sind. Solche Krankheitsbilder sind z. B. arterielle Thrombosen bei Endothelläsionen, Atherosklerose, hämostatische arterielle und venöse Thrombosen sowie der Myokardinfarkt. Aufgrund ihrer Wirkung auf den Blutdruck kommen die Verbindungen der Formel I auch zur Behandlung von pulmonalem sowie systemischem Hochdruck in Betracht. Vorteilhaft sind die erfindungsgemäßen Substanzen auch zur Verminderung der Aggregabilität bei extrakorporalen Blutkreisläufen (künstliche Niere, Herz-Lungen-Maschine etc.) einsetzbar, wobei man die Substanzen in mikromolaren Konzentrationen dem Patientenblut zusetzt.

Die Erfindung betrifft dementsprechend auch Arzneimittel, die als Wirkstoff eine oder mehrere der erfindungsgemäßen heterocyclischen Verbindungen enthalten. Der Wirkstoffgehalt pro Einzeldosis beträgt dabei 0,01-10 mg, und zwar bei Zubereitungsformen für die parenterale Applikation 0,01-1 mg und bei Zubereitungen für die orale Applikation 0,1-10 mg. Arzneimittel zur parenteralen Applikation können sowohl Lösungen als auch Suspensionen darstellen, es kommen aber auch leicht rekonstituierbare Trockenzubereitungen in Betracht, wie z. B. in Einzelabpackung lyophilisierte Natriumsalze der Verbindungen der Formel I.

Für die orale Verabreichung kommen Tabletten, Dragees oder Kapseln in Betracht, wobei die Wirkstoffe gegebenenfalls mit üblichen Trägermaterialien, Tablettensprengmitteln, Bindemitteln usw. vermischt bzw. verarbeitet sind. Solche oralen Verabreichungsformen können auch in üblicher Weise so hergestellt werden, daß sie den Wirkstoff verzögert freisetzen, um so über einen längeren Zeitraum eine gleichförmige Versorgung des Patienten mit dem Wirkstoff zu gewährleisten. In diesem Fall ist es möglich, die vorstehend genannte Einzeldosis zu erhöhen, beispielsweise auf 25 oder 50 mg pro Einzelform der Retardzubereitung, vorausgesetzt, daß die Freigaberate so ist, daß die Blutspiegel an Wirkstoffen nicht höher liegen als bei oraler Verabreichung einer nicht-retardierten Zubereitungsform.

Die erfindungsgemäße Herstellung dieser Arzneimittel erfolgt in an sich bekannter Weise, wobei selbstverständlich bei der Herstellung der parenteral anzuwendenden Zubereitungen auf Sterilität und — wenn sie in flüssiger Form vorliegen — Isotonie zu achten ist.

Die Erfindung bezieht sich weiterhin auf die Herstellung der Verbindungen der Formel I. Dazu wird zunächst ein Lactol der Formel, das gegebenenfalls in der 15S-Form vorliegt,

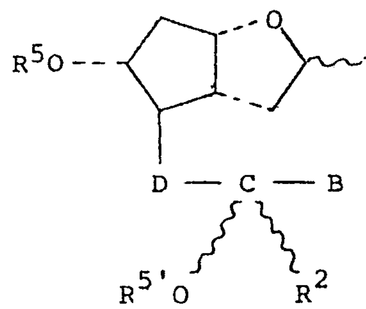

(II)

in der B und $R^2$ die gleiche Bedeutung wie in Formel I haben, D eine Gruppe der Formeln $—CH_2—CH_2—$, (trans)-CH = CH— oder CH = CHal—, in der Hal für Chlor, Brom oder Jod steht, darstellt, und worin $R^5$ und $R^{5'}$ gleich oder verschieden sind und jeweils für Wasserstoff oder für eine unter milden Bedingungen abspaltbare Schutzgruppe wie z. B. eine Tetrahydropyranylgruppe (THP) oder eine Trimethylsilylgruppe (TMS) stehen, mit einem Phosphoniumsalz der Formel

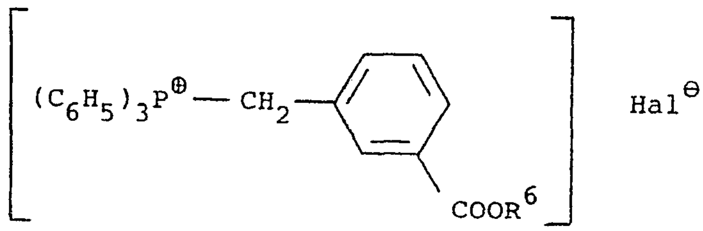

(III)

in der $R^6$ Wasserstoff oder einen Alkylrest mit 1-6 Kohlenstoffatomen, vorzugsweise Methyl oder Athyl darstellt und Hal ein Chlor-, Brom- oder Jodatom, vorzugsweise ein Bromatom, bedeutet, in einem eine starke Base enthaltenden Lösungsmittel bei 10-30 °C unter Sauerstoffausschluß umgesetzt und, wenn $R^6$ für Wasserstoff steht, die Carboxylgruppe durch Behandeln mit Diazomethan in Äther bei ca. 0-30 °C in die Methylestergruppe überführt und schließlich, werden, wenn $R^5$ bzw. $R^{5'}$ Schutzgruppen darstellen, diese unter schonenden Bedingungen abgespalten. $R^6$ kann dabei ein geradkettiger oder verzweigter Alkylrest sein, vorzugsweise stellt er Methyl oder Äthyl dar.

Als bei der Kondensation zuzusetzende starke Base kommen insbesondere Butyllithium, Natriumhydrid oder Kalium-tert.-butylat in Betracht, wobei man in Lösungsmitteln wie z. B. Dimethylsulfoxid, Äther, Tetrahydrofuran oder Dimethylformamid arbeitet.

Die Verbindung der Formel III wird in der ein- bis sechsfachen molaren Menge bezogen auf die Verbindung der Formel II eingesetzt, wobei 2 oder mehr Mol Anwendung finden, wenn D eine —CH = CHal-Gruppe darstellt, da dann unter den Reaktionsbedingungen unter Abspaltung von Halogenwasserstoff diese Gruppe in die —C ≡ C-Gruppe übergeht.

Zur Fernhaltung von Sauerstoff haben sich insbesondere Schutzgase wie Argon oder Stickstoff bewährt. Die Reaktion dauert z. B. 1-12 Stunden und kann in üblicher Weise hinsichtlich ihres Ablaufes chromatographisch verfolgt werden.

Zweckmäßig wird dem Reaktionsgemisch eine katalytische Menge einer Carbonsäure zugesetzt. Hierfür eignen sich beispielsweise niedere aliphatische Carbonsäuren, wie Ameisensäure, Essigsäure, Propionsäure, Bromessigsäure, Trichloressigsäure oder Trifluoressigsäure, aber auch beispielsweise Phenylessigsäure, p-Nitrobenzoesäure, p-Chlorbenzoesäure oder p-Methoxybenzoesäure und insbesondere die unsubstituierte Benzoesäure.

Die Abspaltung der gegebenenfalls vorhandenen Schutzgruppen $R^5$ bzw. $R^{5'}$ kann zum Beispiel mit Tetrahydrofuran/Eisessig/Wasser im Volumenverhältnis 1 : 3 : 1 in einem Zeitraum von 1 bis 5 Stunden bei 30 bis 50 °C erfolgen.

Man erhält so eine Verbindung der Formel IV

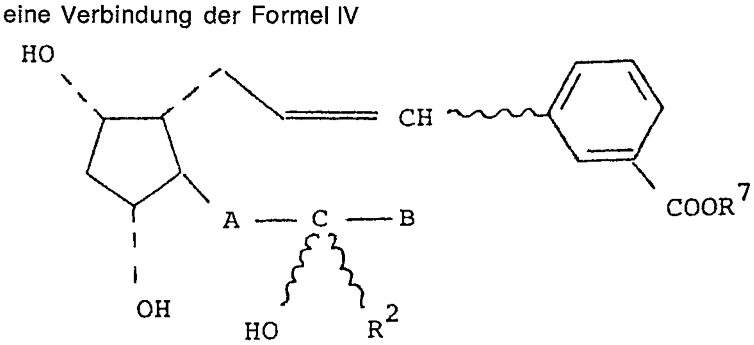

(IV)

in der $R^2$, A und B die gleiche Bedeutung wie in der Formel I haben und $R^7$ einen Alkylrest mit 1-6 Kohlenstoffatomen bedeutet.

Diese kann gewünschtenfalls z. B. durch Säulenchromatographie an Kieselgel in die 15R- bzw. 15S-Formen aufgetrennt werden. Die so erhaltenen Produkte der Formel IV werden in an sich bekannter Weise mit einem elektrophilen Reagenz wie zum Beispiel Jod, Jod-Jodkalium oder N-Bromsuccinimid in einem inerten Lösungsmittel, gegebenenfalls in Gegenwart von Wasser unter Zusatz eines säurebindenden Mittels bei etwa 0 °C-30 °C zu einer Verbindung der Formel V

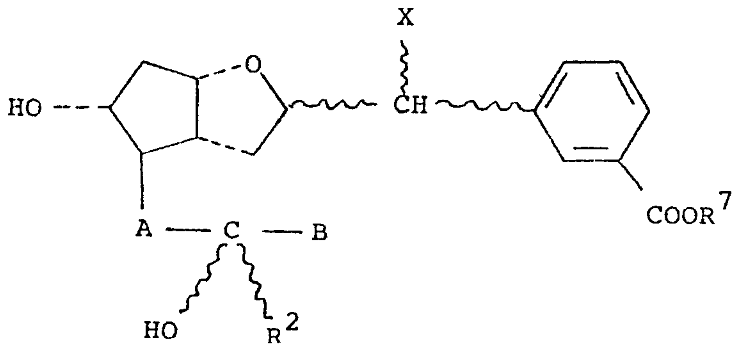

(V)

in der $R^2$, $R^7$, A und B die gleiche Bedeutung wie oben haben und X für ein Brom- oder ein Jodatum steht, halogeniert und cyclisiert. Als Lösungsmittel für diese Reaktion kommen beispielsweise Äthyläther, Tetrahydrofuran, Dichlormethan, Chloroform oder Gemische aus diesen Lösungsmitteln in Betracht, wobei die Reaktion innerhalb von etwa 30 Minuten bis 12 Stunden (der Ablauf der Reaktion ist in üblicher Weise durch chromatographische Analyse feststellbar) abläuft. Als säurebindende Mittel kommen Substanzen wie beispielsweise Natriumhydrogencarbonat, Natriumcarbonat, Kaliumcarbonat, Magnesiumoxyd oder Calciumcarbonat in Betracht.

Vorzugsweise bedeuten X ein Jodatom und $R^7$ eine Methyl- oder Äthylgruppe.

Behandelt man die Verbindung der Formel V mit der 1- bis 5-fachen molaren Menge einer Base, so wird bei Temperaturen von 0-90 °C innerhalb von etwa 4-12 Stunden Halogenwasserstoff abgespalten, wobei eine Verbindung der Formel Ia

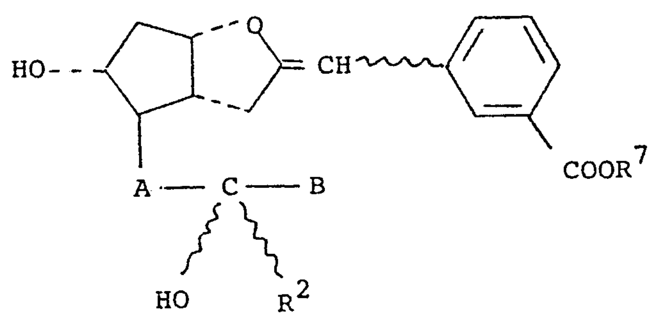

(Ia)

in der $R^2$, $R^7$, A und B die vorstehend genannte Bedeutung haben, entsteht und die gegebenenfalls durch Verseifung der Gruppierung $COOR^7$ in die Verbindung der Formel I übergeführt werden kann, in der $R^1$ für Wasserstoff oder ein Kation steht. Vorzugsweise erfolgt die Umsetzung der verbindung der Formel V mit der Base bei Temperaturen von 0-30 °C. Für die Abspaltung des Halogenwasserstoffes (HX) aus der Verbindung der Formel V eignen sich als Basen insbesondere Natriumhydroxyd, Kaliumhydroxyd, Natriummethylat, Natriumäthylat, Kalium-tert.-butylat sowie Triäthylamin, Dicyclohexylamin, 1,5-Diazabicyclo-(4.3.0)-non-5-en (DBN) oder 1,5-Diazabicyclo-(5.4.0)-undec-5-en (DBU) usw. Diese Abspaltungsreaktion kann in Abwesenheit oder auch in Gegenwart eines Lösungsmittels wie z. B. Toluol, Methanol oder Äthanol durchgeführt werden.

Die Verseifung der Estergruppe $COOR^7$ erfolgt zweckmäßig in wässrig alkoholischer Lösung wie z. B. wasserhaltigem Methanol oder wasserhaltigem Äthanol unter Zusatz der (bezogen auf die Substanz der Formel Ia) 1- bis 5-fachen molaren Menge an Natrium- oder Kaliumhydroxyd bei 10 bis 50 °C. Die Reaktion dauert ca. 6-48 Stunden, wobei es auch möglich ist, die Abspaltung von Halogenwasserstoff und die Verseifung der Estergruppe im gleichen Reaktionsgefäß ohne intermediäre Isolierung der Verbindung der Formel Ia durchzuführen.

Die bei der Verseifung der Formel Ia erhaltenen Verbindungen der Formel I, in denen $R^1$ ein Kation darstellt, können — gegebenenfalls nach Auftrennung in die 5E- und 5Z-Isomeren mit Hilfe der Hochdruckflüssigkeitschromatographie (HPLC) unter « reversed-phase »-Bedingungen — gewünschtenfalls in üblicher Weise (zum Beispiel mit Ionenaustauschchromatographie) in andere Salze übergeführt werden.

Die Ausgangsverbindungen der Formel II bzw. III werden entsprechend Literaturangaben bzw. in analoger Weise dazu wie folgt erhalten :

A) Phosphoniumsalze (Verbindungen der Formel III)

Stöchiometrische Mengen des betreffenden Aralkylhalogenides und Triphenylphosphin werden 1 1/2 Stunden in Acetonitril am Rückfluß gekocht und dann am Rotationsverdampfer unter Erwärmen auf 50-70 °C im Vakuum eingeengt. Der feste Rückstand wird gründlich mit kaltem Aceton gewaschen und, wenn erforderlich, aus Wasser umkristallisiert. Es wurde so z. B. das m-Carboxyphenyl-methyl-triphenylphosphoniumbromid vom Schmelzpunkt 256-258 °C erhalten.

B) 3α,5α-Dihydroxy-2β-[(3RS)-3-hydroxy-3-methyl-trans-1-octenyl]-cyclopentan-1α-acetaldehyd-γ-lactol-3-trimethylsilyläther wurde nach E. W. Yankee et al. J.A.C.S. *96* 5865 (1974) hergestellt.

C) Die Darstellung der als Ausgangsmaterialien erforderlichen Lactole der Formel II wird in folgenden Veröffentlichungen beschrieben oder kann analog erfolgen :

| | |
|---|---|
| 15-Alkyl | E. W. Yankee et al. J.A.C.S. *96* 5865 ff (1974) |
| 15-H | E. J. Corey et al. J.A.C.S. *91* 5675 (1969) |
| 13,14-Dihydro | DE-OS 23 55 540 |
| 13,14-Dehydro | BE-PS 832 891 |
| 16-Alkyl | DE-OS 22 17 044 |
| 16,16-Dialkyl | DE-OS 22 17 044 |
| 15(4'-Alkyl-cyclohexyl) | BE-PS 782 822 |

Die folgenden Beispiele dienen zur näheren Erläuterung der Erfindung. Alle Temperaturangaben darin sind unkorrigiert. Die Kernresonanzspektren wurden (Protonenspektren bei 60 MHz) mit einem handelsüblichen Gerät gemessen. Die $R_f$-Werte wurden dünnschichtchromatographisch ermittelt.

## Beispiel 1

a) [(5EZ, 13E, 9α, 11α, 15RS)-2,3,4-Trinor-1,5-inter-m-phenylen-9,11,15-trihydroxy-15-methyl]-prosta-5,13-diensäure.

1,8 ml Butyllithium (15 %-ige Lösung in Hexan) werden unter Stickstoff und Feuchtigkeitsausschluß bei Raumtemperatur tropfenweise zu 25 ml trockenem Dimethylsulfoxid gegeben und 30 Minuten gerührt. Anschließend wird tropfenweise eine Lösung von 670 mg m-Carboxyphenyl-methyl-triphenylphosphoniumbromid (allgemeine Formel III : $R^6 = H$) und 67 mg Benzoesäure in 10 ml trockenem Dimethylsulfoxid zugegeben.

Nach 15 Minuten wird tropfenweise eine Lösung von 500 mg 3α,5α-Dihydroxy-2β-[(3RS)-3-hydroxy-3-methyl-trans-1-octenyl]-cyclopentan-1α-acetaldehyd-γ-lactol-3-trimethylsilyläther in 5 ml Dimethylsulfoxid zugefügt. Das Reaktionsgemisch wird 12 Stunden bei Raumtemperatur gerührt, dann in gesättigte Kochsalzlösung gegeben und dreimal mit Essigester extrahiert. Die organische Phase wird verworfen. Die wässrige Phase wird mit verdünnter Salzsäure auf pH 4 angesäuert und mehrmals gründlich mit Essigsäureäthylester extrahiert. Die gesammelten organischen Phasen werden mit Wasser gewaschen, über Magnesiumsulfat getrocknet und am Rotationsverdampfer im Vacuum bei 40 °C eingeengt.

Das Rohprodukt (520 mg) ergibt nach Säulenchromatographie an Kieselgel mit Essigsäureäthylester-Eisessig 99,5 : 0,5 300 mg eines schwachgelben Öls.

$R_f = 0,125$ (Toluol-Dioxan-Eisessig 68 : 32 : 0,5).

Angabe der chemischen Verschiebung in ppm

$^1$H-NMR($CDCl_3$) : (0,87,t,3H) ; (1,27,s,3H) ; (3,63-4,36,m,5H) ; (5,36-5,6,m,2H) ; (5,6-6,5,m,2H) ; (7,2-7,5, m,2H) ; (7,5-8,03,m,2H).

$^{13}$C-NMR (Methanol-$d_4$) : 27.271 (15S-Me) ; 28.184 (15R-Me)

b) [(5EZ, 13E, 9α, 11α, 15RS)-2,3,4-Trinor-1,5-inter-m-phenylen-9,11,15-trihydroxy-15-methyl]-prosta-5,13-diensäuremethylester. Allgemeine Formel IV : $R^2 = CH_3$ : $R^7 = CH_3$ ; A = trans-CH = CH— ; B = n-$C_5H_{11}$.

370 mg der in Beispiel 1a dargestellten Verbindung werden in 50 ml Diäthyläther gelöst und unter Rühren bei Raumtemperatur mit ätherischer Diazomethanlösung bis zur Beendigung der Gasentwikklung versetzt. Anschließend wird das gelbe Reaktionsgemisch mit wässriger Natriumhydrogencarbonatlösung gerührt. Die farblose Ätherphase wird abgetrennt, über Magnesiumsulfat getrocknet und am Rotationsverdampfer bei 30° im Vakuum eingeengt. Das zurückbleibende schwachgelbe Öl (350 mg) zeigt dünnschichtchromatographisch zwei Hauptflecke $R_f$ 0,48 bzw. 0,55 (Diäthyläther-Acetonitril 3 : 2). Säulenchromatographie des Rohproduktes an Kieselgel mit Diäthyläther-Acetonitril 3 : 2 liefert zwei isomere Verbindungen, die das 15R- und das 15S-Isomer darstellen, von denen das erstere verworfen wird.

Das 15S-Isomer wird in einer Ausbeute von 130 mg erhalten. Sein $R_f$-Wert beträgt 0,48 (Diäthyläther-Acetonitril 3 : 2).

$^1$H-NMR ($CDCl_3$) : (0,87,t,3H) ; (1,27,s,3H) ; (3,7-4,43,m,3,9,s,5H) ; (5,37-5,6,m,2H) ; (5,6-6,73,m,2H) ; (7,2-7,5,m,2H) ; (7,67-8,m,2H).

$^{13}$C-NMR (Methanol-$d_4$) : 27.454

c) [(13E, 5RS, 6RS, 9α, 11α, 15S)-2,3,4-Trinor-1,5-inter-m-phenylen-5-iodo-6,9-epoxy-11,15-dihydroxy-15-methyl]-prost-13-ensäuremethylester.

(Allgemeine Formel V : $R^2 = R^7 = CH_3$, X = J, A = trans-CH = CH—, B = n-$C_5H_{11}$)

Zu einer Lösung von 720 mg Jod in 6 ml Diäthyläther und 2 ml gesättigter, wässriger Natriumhydrogencarbonatlösung wird unter Rühren eine Lösung von 170 mg der in Beispiel 1b dargestellten Verbindung in 2 ml Diäthyläther bei Raumtemperatur im Dunkeln rasch zugegeben.

Das Reaktionsgemisch wird 45 min. im Dunkeln bei Raumtemperatur gerührt und dann mit Natriumthiosulfatlösung behandelt. Das farblose Reaktionsgemisch wird in eiskalte gesättigte Kochsalzlösung gegeben und dreimal gründlich mit je 20 ml Diäthyläther ausgeschüttelt.

Die Ätherphasen werden über Magnesiumsulfat getrocknet, filtriert und am Rotationsverdampfer kalt im Dunkeln unter Vakuum eingeengt. Das als gelbliches Öl anfallende Rohprodukt (180 mg, $R_f$ = 0,58 Essigsäureäthylester) wird ohne weitere Reinigung verarbeitet.

d) [(5EZ, 13E, 9α, 11α, 15S)-2,3,4-Trinor-1,5-inter-m-phenylen-6,9-epoxy-11,15-dihydroxy-15-methyl]-prosta-5,13-diensäuremethylester.

(Allgemeine Formel I : $R^1 = R^2 = CH_3$ ; A = trans-(CH = CH) ; B = n-$C_5H_{11}$)

180 mg der in Beispiel 1c dargestellten Verbindung werden in 2 ml Toluol gelöst, mit 100 μl DBN versetzt und im Dunkeln 4 1/2 Stunden gerührt.

Das Toluol wird mit Stickstoff bei Raumtemperatur abgeblasen. Der Rückstand liefert nach Säulenchromatographie an Kieselgel mit Essigsäureäthylester 90 mg eines schwachgelben Öls, das aus 2 Komponenten besteht.

$R_f$ = 0,55 und 0,6 (Essigsäureäthylester)

e) Natriumsalz der [(5EZ, 13E, 9α, 11α, 15S)-2,3,4-Trinor-1,5-inter-m-phenylen-6,9-epoxy-11,15-dihydroxy-15-methyl]-prosta-5,13-diensäure.

(Allgemeine Formel I : $R^1$ = Na ; $R^2 = CH_3$ ; A = trans-(CH = CH) ; B = n-$C_5H_{11}$)

90 mg der in Beispiel 1d dargestellten Verbindung werden in 1 ml Methanol gelöst, mit 600 μl 1 n-NaOH versetzt und bei 30-40° 12 Stunden gerührt.

$R_f$ : 0,36 und 0,44 (Kieselgel RP-8, Methanol-Wasser 80 : 20). Hochdruckflüssigkeitschromatographie mit Methanol-Wasser 40 : 60 an Kieselgel RP-18 liefert nach anschließender Gefriertrocknung :

i) Natriumsalz der [(5Z, 13E, 9α, 11α, 15S)-2,3,4-Trinor-1,5-inter-m-phenylen-6,9-epoxy-11,15-dihydroxy-15-methyl]-prosta-5,13-diensäure.

Ausbeute 35 mg

$R_f$ = 0,36 (Kieselgel RP-8, Methanol-Wasser 80 : 20)

$^1$H-NMR (Methanol-$d_4$) : (0,9,t,3H) ; (1,28,s,3H) ; (3,63-4,2,m,1H) ; (5,28,s verbreitert, 1H) ; (5,5-5,73,m, 2H) ; (7,06-7,48,m,1H) ; (7,53-7,8,m,2H) ; (7,96-8,1,m,1H).

ii) Natriumsalz der [(5E, 13E, 9α, 11α, 15S)-2,3,4-Trinor-1,5-inter-m-phenylen-6,9-epoxy-11,15-dihydroxy-15-methyl]-prosta-5,13-diensäure.

Ausbeute 18 mg $R_f$ 0,44 (Kieselgel RP-8, Methanol-Wasser 80 : 20)

$^1$H-NMR (Methanol-$d_4$) : (0,9,t,3H) ; (1,3,s,3H) ; (3,56-4,16,m,1H) ; (5,5-5,7,m,2H) ; (5,9,s verbreitert, 1H) ; (7,13-7,48,m,2H) ; (7,53-7,9,m,2H).

(Bei den oben erwähnten Kieselgelen RP-8 bzw. RP-18 handelt es sich um Handelsprodukte der Firma Merck AG, Darmstadt).

## Beispiel 2

Unter Benutzung des in Beispiel 1 beschriebenen Vorgehens erhält man aus den entsprechenden Ausgangsmaterialien :

a) [(5E, 13E, 9α, 11α, 15S)-2,3,4-Trinor-1,5-inter-m-phenylen-6,9-epoxy-11,15-dihydroxy]-prosta-5,13-diensäure Natrium-Salz

(Allgemeine Formel I : $R^1$ = Na ; $R^2$ = Wasserstoff ; A = trans-(CH = CH)— ; B = n-$C_5H_{11}$)

$^1$H-NMR (Methanol-$d_4$) : (0,9,t,3H) ; (1,3,s,3H) ; (3,6-4,15,m,2H) ; (5,5-5,7,m,2H) ; (5,85-6,05,s verbreitert, 1H) ; (7,15-7,5,m,2H) ; (7,5-8,m,2H).

$R_f$ = 0,44 (Kieselgel RP-8) Methanol-Wasser 80 : 20.

b) [(5Z, 13E, 9α, 11α, 15S)-2,3,4-Trinor-1,5-inter-m-phenylen-6,9-epoxy-11,15-dihydroxy]-prosta-5,13-diensäure Natrium-Salz

(Allgemeine Formel I : $R^1$ = Na ; $R^2$ = Wasserstoff ; A = trans-(CH = CH)— ; B = n-$C_5H_{11}$)

$^1$H-NMR (Methanol-$d_4$) : (0,9,t,3H) ; (3,5-4,1,m,3H) ; (5,25,s verbreitert, 1H) ; (5,45-5,7,m,2H) ; (7-7,5,m, 1H) ; (7,5-7,85,m,2H) ; (8,s verbreitert, 1H)

$R_f$ = 0,34 (Kieselgel RP-8) Methanol-Wasser 80 : 20

## Beispiel 3

Analog zu dem in Beispiel 1 beschriebenen Vorgehen erhält man aus m-Carboxyphenyl-methyl-triphenylphosphoniumbromid und der entsprechenden Verbindung der Formel II :

a) [(5E, 13E, 9α, 11α, 15S)-2,3,4-Trinor-1,5-inter-m-phenylen-6,9-epoxy-11,15-dihydroxy-15-cyclohexyl-16,17,18,19,20-pentanor]-prosta-5,13-diensäure Natrium-Salz

(Allgemeine Formel I : $R^1$ = Na ; $R^2$ = Wasserstoff ; A = trans-(CH = CH)— ; 8 = Cyclohexyl)

$^1$H-NMR (Methanol-$d_4$) : (3,65-4,1,m,3H) ; (5,5-5,7,m,2H) ; (5,97,s verbreitert, 1H) ; (7,2-7,5,m,2H) ; (7,5-8,m,2H)

$R_f$ = 0,52 (Kieselgel RP-8) Methanol-Wasser 80 : 20.

b) [(5Z, 13E, 9α, 11α, 15S)-2,3,4-Trinor-1,5-inter-m-phenylen-6,9-epoxy-11,15-dihydroxy-15-cyclohexyl-16,17,18,19, 20-pentanor]-prosta-5,13-diensäure Natrium-Salz

(Allgemeine Formel I : $R^1$ = Na ; $R^2$ = Wasserstoff ; A = trans-(CH = CH)— ; B = Cyclohexyl)

$^1$H-NMR (Methanol-$d_4$) : (3,67-3,95,m,2H) ; (4-4,2,m,2H) ; (5,37,s verbreitert, 1H) ; (5,5-5,7,m,2H) ; (7,15-7,4,m,1H) ; (7,6-8,15,m,2H).

$R_f$ = 0,38 (Kieselgel RP-8) Methanol-Wasser 80 : 20

## Beispiel 4

Man geht entsprechend dem in den vorhergehenden Beispielen angewendetem Verfahren vor und erhält :

a) [(5E, 13E, 9α, 11α, 15S, 16RS)-2,3,4-Trinor-1,5-inter-m-phenylen-6,9-epoxy-11,15-dihydroxy-16-ethyl)-prosta-5,13-diensäure Natriumsalz

(Allgemeine Formel I : $R^1$ = Na ; $R^2$ = Wasserstoff ; A = trans-(CH = CH) ; B = —C($R^3$, $R^4$)—$(CH_2)_3$—$CH_3$ darin : $R^3$ = $C_2H_5$, $R^4$ = Wasserstoff)

$^1$H-NMR (Methanol-$d_4$) : (0,9,t,6H) ; (3,6-4,2,m,3H) ; (5,5-5,7,m,2H) ; (5,95,s verbreitert, 1H) ; (7,15-7,35, m,2H) ; (7,6-7,95,m,2H).

$R_f$ = 0,68 (Kieselgel RP-8) Methanol-Wasser 80 : 20

b) [(5Z, 13E, 9α, 11α, 15S, 16RS)-2,3,4-Trinor-1,5-inter-m-phenylen-6,9-epoxy-11,15-dihydroxy-16-ethyl]-prosta-5,13-diensäure Natriumsalz

(Allgemeine Formel I : $R^1$ = Na ; $R^2$ = Wasserstoff ; A = trans-(CH = CH)— ; B = —CH($C_2H_5$)—$(CH_2)_3$—$CH_3$

$^1$H—NMR (Methanol-$d_4$) : (0,9,t,6H) ; (3,75-4,55,m,3H) ; (5,35,s verbreitert, 1H) ; (5,5-5,7,m,2H) ; (7,1-7,4, m,1H) ; (7,55-7,95,m,1H) ; (8,05,m,1H)

$R_f$ = 0,63 (Kieselgel RP-8) Methanol-Wasser 80 : 20

## Beispiel 5

Ausgehend von m-Carboxyphenylmethyl-triphenylphosphoniumbromid und 3α, 5α-Dihydroxy-2β-[(3RS)-3-hydroxy-1-octyl]-cyclopentan-1α-acetaldehyd-γ-lactol-bis-tetrahydropyranyläther erhält man entsprechend dem in Beispiel 1 beschriebenen Vorgehen :

a) [(5E, 9α, 11α, 15RS)-2,3,4-Trinor-1,5-inter-m-phenylen-6,9-epoxy-11,15-dihydroxy]-prosta-5-en-säure Natriumsalz

(Allgemeine Formel I : $R^1$ = Na ; $R^2$ = Wasserstoff ; A = —$CH_2$—$CH_2$— ; B = n-$C_5H_{11}$)

$^1$H-NMR : (Methanol-$d_4$) : (0,9,t,3H) ; (5,9,s verbreitert, 1H) ; (7,18-8,15,m,4H) ; Kein Signal bei 5,5-5,7

$R_f$ = 0,48 (Kieselgel RP-8) Methanol-Wasser 80 : 20

b) [(5Z, 9α, 11α, 15RS)-2,3,4-Trinor-1,5-inter-m-phenylen-6,9-epoxy-11,15-dihydroxy]-prosta-5-en-säure Natriumsalz

(Allgemeine Formel I : $R^1$ = Na ; $R^2$ = Wasserstoff ; A = —$CH_2$—$CH_2$— ; B = n-$C_5H_{11}$)

$^1$H-NMR (Methanol-$d_4$) : (0,9,t,3H) ; (5,3,s verbreitert, 1H) ; (7,1-8,05,m,4H) ; Kein Signal bei 5,5-5,7

$R_f$ = 0,41 (Kieselgel RP-8) Methanol-Wasser 80 : 20

Mit Hilfe der oben beschriebenen Verfahrensweisen, insbesondere entsprechend dem aus dem Beispiel 1 ersichtlichen Vorgehen, lassen sich auch folgende Verbindungen der Formel I erhalten :

[(5EZ, 13E, 9α, 11α, 15S)-2,3,4-Trinor-1,5-inter-m-phenylen-6,9-epoxy-11,15-dihydroxy]-prosta-5,13-diensäure Kaliumsalz ;

[(5EZ, 13E, 9α, 11α, 15S)-2,3,4-Trinor-1,5-inter-m-phenylen-6,9-epoxy-11,15-dihydroxy-16,16-dimethyl]-prosta-5,13-diensäure Natriumsalz ;

[(5EZ, 13E, 9α, 11α, 15S, 16RS)-2,3,4-Trinor-1,5-inter-m-phenylen-6,9-epoxy-11,15-dihydroxy-16-äthyl]-prosta-5,13-diensäure Kaliumsalz ;

[(5EZ, 13E, 9α, 11α, 15S)-2,3,4-Trinor-1,5-inter-m-phenylen-6,9-epoxy-11,15-dihydroxy-15-cyclohexyl-16,17,18,19,20-pentanor]-prosta-5,13-diensäure Kaliumsalz ;

[(5EZ, 13E, 9α, 11α, 15S, 4'-trans)-2,3,4-Trinor-1,5-inter-m-phenylen-6,9-epoxy-11,15-dihydroxy-15-(4'-methyl-cyclohexyl)-16,17,18,19,20-pentanor]-prosta-5,13-diensäure Natriumsalz ;

[(5EZ, 13E, 9α, 11α, 15S, 4'-trans)-2,3,4-Trinor-1,5-inter-m-phenylen-6,9-epoxy-11,15-dihydroxy-15-(4'-äthyl-cyclohexyl)-16,17,18,19,20-pentanor]-prosta-5-13-diensäure Kaliumsalz ;

[(5EZ, 9α, 11α, 15S)-2,3,4-Trinor-1,5-inter-m-phenylen-6,9-epoxy-11,15-dihydroxy]-prosta-5-en-13-in-säure Natriumsalz ;

[(5EZ, 9α, 11α, 15S)-2,3,4-Trinor-1,5-inter-m-phenylen-6,9-epoxy-11,15-dihydroxy-15-cyclohexyl-16,17,18,19,20-pentanor]-prosta-5-ensäure Natriumsalz ;

[(5EZ, 9α, 11α, 15S)-2,3,4-Trinor-1,5-inter-m-phenylen-6,9-epoxy-11,15-dihydroxy-15-cyclohexyl-16,17,18,19,20-pentanor]-prosta-5-en-13-in-säure Natriumsalz ;

[(5EZ, 13E, 9α, 11α, 15S)-2,3,4-Trinor-1,5-inter-m-phenylen-6,9-epoxy-11,15-dihydroxy-15-methyl-15-cyclohexyl-16,17,18,19,20-pentanor]-prosta-5,13-diensäure Natriumsalz.

**Ansprüche** (für die Vertragsstaaten : BE, CH, DE, FR, GB, IT, LI, LU, NL, SE)

1. Heterocyclische Verbindungen der Formel

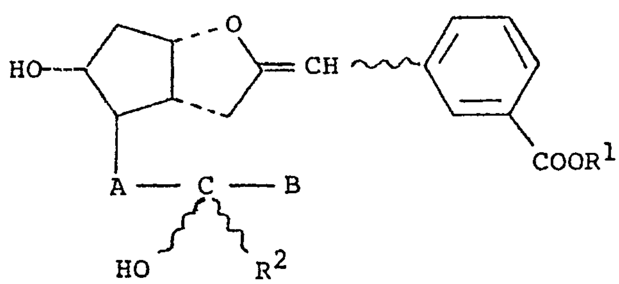

(I)

worin der Phenylrest in Bezug auf die Doppelbindung E- und/oder Z-Konfiguration aufweisen und am (unter anderem den Rest $R^2$ tragenden) Kohlenstoffatom 15 RS- oder S-Konfiguration vorliegen kann und worin $R^1$ ein Wasserstoffatom, ein pharmazeutisch verträgliches Kation oder einen geradkettigen oder verzweigten Alkylrest mit 1-6 Kohlenstoffatomen bedeutet, $R^2$ für Wasserstoff oder Methyl steht, A eine der Gruppen $-CH_2-CH_2-$, (trans)-CH = CH— oder —C ≡ C— ist und B einen Alkylrest mit 5 bis 9 Kohlenstoffatomen der Struktur

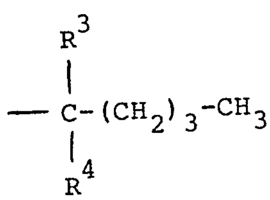

in der $R^3$ und $R^4$ gleich oder verschieden sind und für Wasserstoff, Methyl oder Äthyl stehen, oder einen Cyclohexylrest, der in der 4'-Stellung durch einen Methyl- oder Äthylrest substituiert sein kann, bedeutet.

2. Heterocyclische Verbindungen nach Anspruch 1 entsprechend der Formel

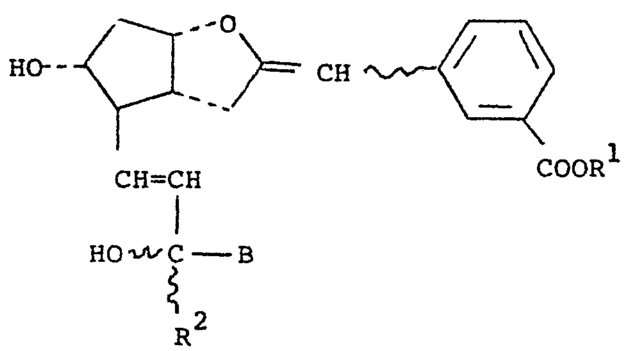

worin $R^1$, $R^2$ und B die gleiche Bedeutung wie in Formel I haben.

3. Heterocyclische Verbindungen nach Anspruch 1 entsprechend der Formel

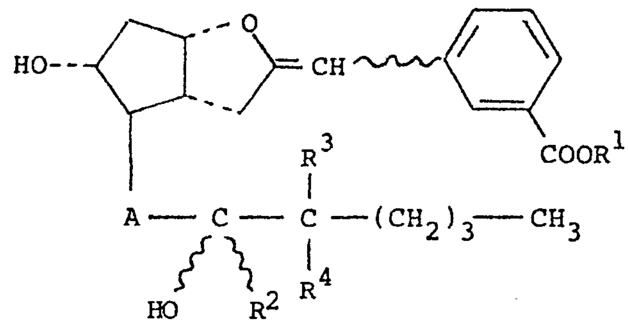

worin $R^1$ bis $R^4$ und A die gleiche Bedeutung wie in Formel I haben.

4. Heterocyclische Verbindungen nach Anspruch 1 entsprechend der Formel

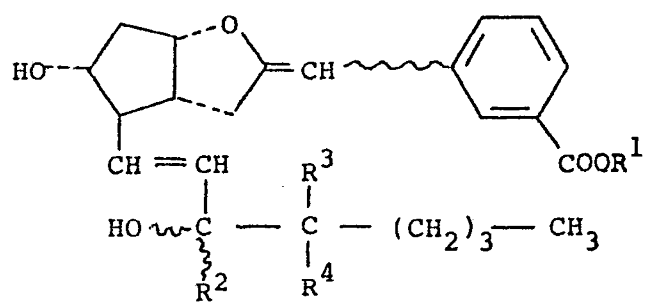

worin $R^1$ bis $R^4$ die gleiche Bedeutung wie in Formel I haben.

5. Heterocyclische Verbindungen gemäß Ansprüchen 1-4, in denen $R^3$ und $R^4$ für Wasserstoff stehen.

6. Heterocyclische Verbindungen gemäß Ansprüchen 1-4, in denen $R^3$ für Wasserstoff und $R^4$ für einen Äthylrest steht.

7. Heterocyclische Verbindungen gemäß Anspruch 1 entsprechend der Formel

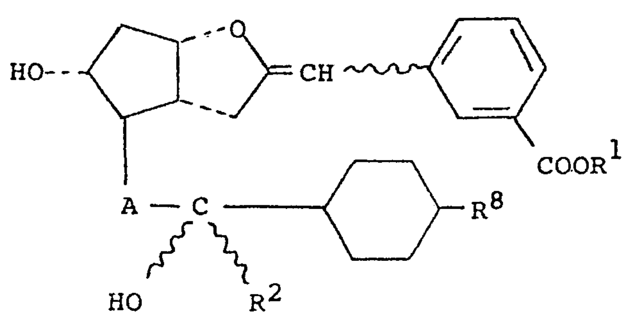

worin $R^1$, $R^2$ und A die gleiche Bedeutung wie in Formel I haben und worin $R^8$ Wasserstoff, Methyl oder Äthyl ist.

8. Heterocyclische Verbindungen gemäß Ansprüchen 1-7, in denen $R^2$ für Wasserstoff steht.

9. Heterocyclische Verbindungen gemäß Anspruch 1 entsprechend der Formel

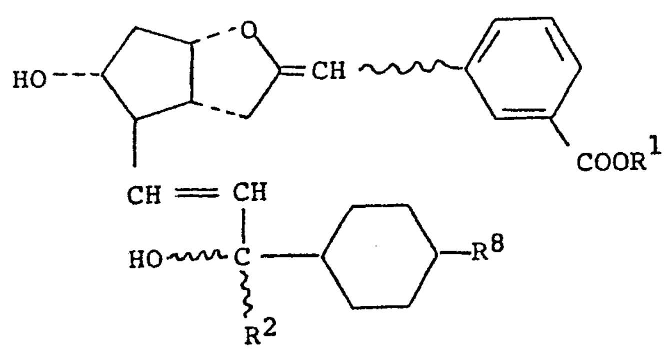

worin $R^1$ und $R^2$ die gleiche Bedeutung wie in Formel I haben und worin $R^8$ Wasserstoff, Methyl oder Äthyl ist.

10. Heterocyclische Verbindungen gemäß Anspruch 1 entsprechend der Formel

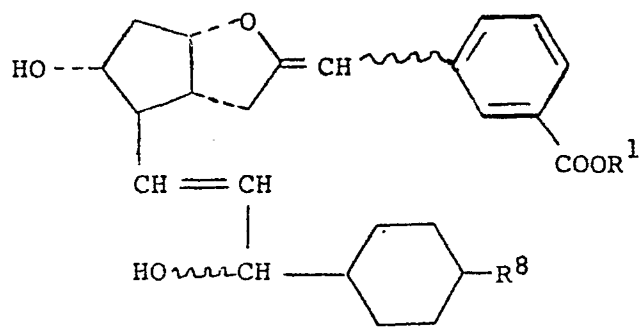

worin $R^1$ die gleiche Bedeutung wie in Formel I hat und worin $R^8$ Wasserstoff, Methyl oder Äthyl ist.

11. Heterocyclische Verbindungen gemäß Ansprüchen 1-10, in denen $R^1$ für einen Methyl- oder einen Äthylrest steht.

12. Heterocyclische Verbindungen gemäß Ansprüchen 1-10, in denen $R^1$ für ein Natrium- oder ein Kaliumion steht.

13. Die 15S-Formen der heterocyclischen Verbindungen gemäß Ansprüchen 1 bis 12.

14. Die heterocyclische Verbindung [(5EZ, 13E, 9α, 11α, 15S)-2,3,4-Trinor-1,5-inter-m-phenylen-6,9-epoxy-11,15-dihydroxy-15-methyl]-prosta-5,13-diensäure und ihre pharmazeutisch verträglichen Salze, nach Anspruch 1 insbesondere ihr Natriumsalz, und ihr Methylester.

15. Die heterocyclische Verbindung [(5EZ, 13E, 9α, 11α, 15S)-2,3,4-Trinor-1,5-inter-m-phenylen-6,9-epoxy-11,15-dihydroxy-15-cyclohexyl-16,17,18,19,20-pentanor]-prosta-5,13-diensäure und ihre pharmazeutisch verträglichen Salze, nach Anspruch 1 insbesondere ihr Natriumsalz, und ihr Methylester.

16. Arzneimittel, dadurch gekennzeichnet, daß es als Wirkstoff eine heterocyclische Verbindung entsprechend den Ansprüchen 1-15 enthält.

17. Arzneimittel gemäß Anspruch 16, dadurch gekennzeichnet, daß es als Wirkstoff 0,01 bis 10 mg einer heterocyclischen Verbindung entsprechend den Ansprüchen 1-15 enthält.

18. Arzneimittel gemäß Ansprüchen 16 und 17, dadurch gekennzeichnet, daß es zur parenteralen Applikation geeignet ist und in einem üblichen flüssigen sterilen Träger 0,01 bis 1 mg einer heterocyclischen Verbindung entsprechend den Ansprüchen 1-15 in gelöster oder suspendierter Form enthält.

19. Arzneimittel gemäß Ansprüchen 16 und 17, dadurch gekennzeichnet, daß es in Form von Tabletten, Dragees oder Kapseln zur oralen Applikation vorliegt und 0,1 bis 10 mg einer heterocyclischen Verbindung entsprechend den Ansprüchen 1-15 sowie übliche Trägermaterialien enthält.

20. Verfahren zur Herstellung von heterocyclischen Verbindungen der Formel

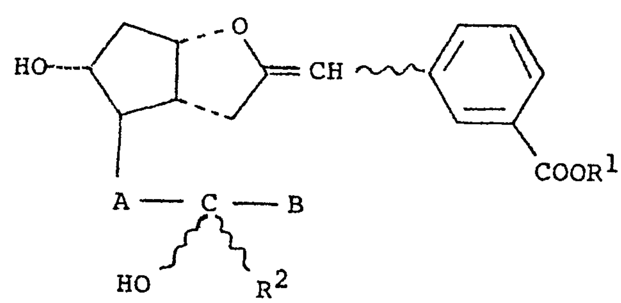

(I)

worin der Phenylrest in Bezug auf die Doppelbindung E- und/oder Z-Konfiguration aufweisen und am (unter anderem den Rest $R^2$ tragenden) Kohlenstoffatom 15 RS- oder S-Konfiguration vorliegen kann, und worin $R^1$ ein Wasserstoffatom, ein pharmazeutisch verträgliches Kation oder einen geradkettigen oder verzweigten Alkylrest mit 1-6 Kohlenstoffatomen bedeutet, $R^2$ für Wasserstoff oder Methyl steht, A eine der Gruppen $-CH_2-CH_2-$, (trans)-CH = CH— oder $-C \equiv C-$ ist und B einen Alkylrest mit 5 bis 9 Kohlenstoffatomen der Struktur

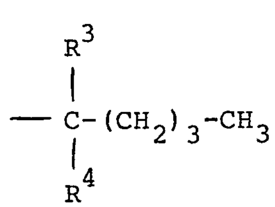

in der $R^3$ und $R^4$ gleich oder verschieden sind und für Wasserstoff, Methyl oder Äthyl stehen, oder einen Cyclohexylrest, der in der 4'-Stellung durch einen Methyl- oder Äthylrest substituiert sein kann, bedeutet, dadurch gekennzeichnet, daß man

a) eine Verbindung der Formel

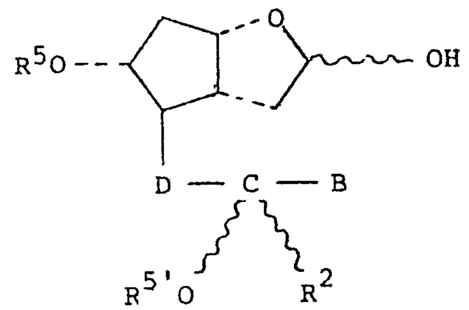

(II)

in der B und $R^2$ die gleiche Bedeutung wie in Formel I haben, D eine $-CH_2-CH_2-$, trans-CH = CH— oder eine —CH = CHal-Gruppe, in der Hal ein Chlor-, Brom- oder Jodatom ist, darstellt und worin $R^5$ und $R^{5'}$ gleich oder verschieden sind und jeweils für ein Wasserstoffatom oder für eine unter milden Bedingungen abspaltbare Schutzgruppe, insbesondere für eine Tetrahydropyranyl- oder eine Trimethylsilylgruppe stehen mit der 1- bis 6-fachen molaren Menge einer Verbindung der Formel

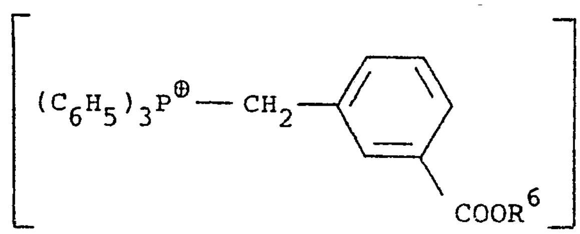

(III)

in der $R^6$ Wasserstoff oder einen Alkylrest mit 1-6 Kohlenstoffatomen, vorzugsweise einen Methyl- oder einen Äthylrest bedeutet und Hal ein Chlor-, Brom- oder Jodatom, vorzugsweise ein Bromatom ist, unter Ausschluß von Sauerstoff bei 10-30 °C in Gegenwart einer starken Base und eines Lösungsmittels umsetzt und gegebenenfalls anschließend, wenn $R^6$ für Wasserstoff steht, die Carboxylgruppe $COOR^6$ mit Diazomethan in Äther bei ca. 0°-30 °C verestert, und, wenn $R^5$ bzw. $R^{5'}$ Schutzgruppen darstellen, diese unter schonenden Bedingungen abspaltet, wobei man eine Verbindung der Formel

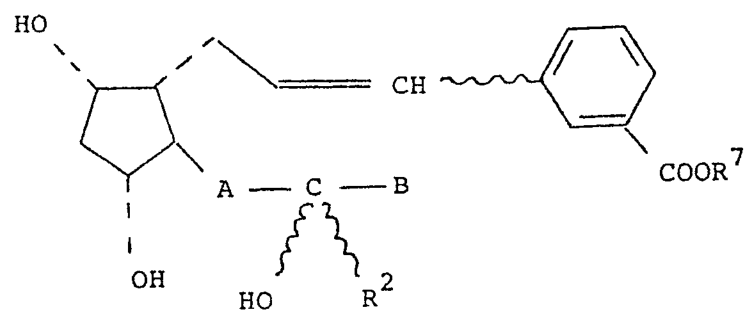

(IV)

in der $R^2$, A und B die gleiche Bedeutung wie in Formel I haben und $R^7$ einen Alkylrest mit 1-6 Kohlenstoffatomen, vorzugsweise einen Methyl- oder einen Äthylrest bedeutet, erhält, diese

b) in Gegenwart eines inerten Lösungsmittels und gegebenenfalls in Gegenwart eines säurebindenden Mittels bei etwa 0°-30 °C halogeniert und zu einer Verbindung der Formel

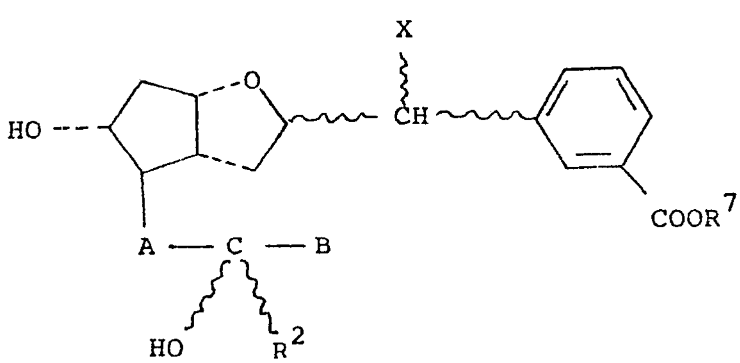

(V)

in der $R^2$, $R^7$, A und B die gleiche Bedeutung wie oben haben und X für Brom oder Jod steht cyclisiert und

c) aus der Verbindung der Formel V bei Temperaturen von etwa 0 bis 90 °C mit der 1- bis 5-fachen molaren Menge einer Base, gegebenenfalls in Anwesenheit eines Lösungsmittels, Halogenwasserstoff HX abspaltet, und gegebenenfalls in der so erhaltenen Verbindung der Formel

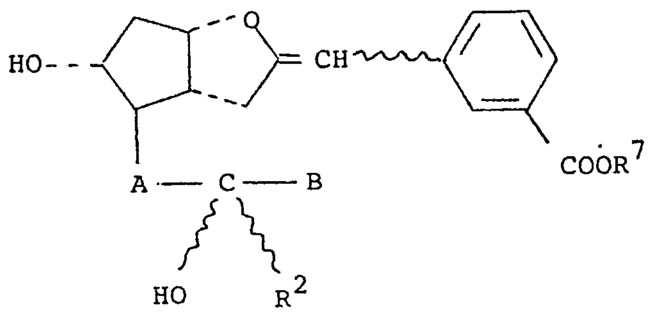

(Ia)

in der $R^2$, $R^7$, A und B die gleiche Bedeutung wie oben haben die Estergruppierung $COOR^7$ in wässrig-alkoholischer Lösung bei etwa 10-50 °C mit einem Alkalihydroxid verseift und vorzugsweise anschließend die so erhaltenen Verbindungen der Formel I in die 5E- und 5Z-Isomeren auftrennt.

21. Verfahren zur Herstellung von heterocyclischen Verbindungen gemäß Anspruch 20, dadurch gekennzeichnet, daß in der Verfahrensstufe a) die Umsetzung der Verbindung der Formel II mit der Verbindung der Formel III unter Zusatz katalytischer Mengen einer Carbonsäure, vorzugsweise von Ameisensäure, Essigsäure, Propionsäure, Bromessigsäure, Trichloressigsäure, Trifluoressigsäure, Phenylessigsäure, p-Nitrobenzoesäure, p-Chlorbenzoesäure, p-Methoxybenzoesäure und insbesondere von Benzoesäure durchgeführt wird.

22. Verfahren zur Herstellung von heterocyclischen Verbindungen gemäß Anspruch 20, dadurch gekennzeichnet, daß in der Verfahrensstufe b zur Halogenierung und Cyclisierung der Verbindung der Formel IV Jod, Jod-Jodkalium oder Bromsuccinimid, gegebenenfalls in Anwesenheit von Wasser und Natriumhydrogencarbonat, Natriumcarbonat, Magnesiumoxid oder Calciumcarbonat, eingesetzt wird.

23. Verfahren zur Herstellung von heterocyclischen Verbindungen gemäß Anspruch 20, dadurch gekennzeichnet, daß in der Verfahrensstufe c die Abspaltung des Halogenwasserstoffes HX mit Hilfe von Natriumhydroxid, Kaliumhydroxid, Natriummethylat, Natriumäthylat, Kalium-tert.-butylat, Triäthylamin, Dicyclohexylamin, 1,5-Diazabicyclo-(4.3.0)-non-5-en oder 1,5-Diazabicyclo-(5.4.0)-undec-5-en erfolgt.

24. Verfahren zur Herstellung von heterocyclischen Verbindungen gemäß Ansprüchen 20 und 23, dadurch gekennzeichnet, daß in der Verfahrensstufe c die Abspaltung des Halogenwasserstoffes HX und die Verseifung der Estergruppe $COOR^7$ ohne intermediäre Isolierung der Verbindung der Formel Ia erfolgt.

25. Verfahren zur Herstellung von heterocyclischen Verbindungen der allgemeinen Formel I in Form der 15S-Isomeren gemäß Anspruch 20, dadurch gekennzeichnet, daß man die RS-Formen der Verbindung der Formel II, IV, V oder Ia bzw. I in an sich bekannter Weise, insbesondere durch Säulenchromatographie, auftrennt.

26. Verfahren gemäß Ansprüchen 20 und 25, dadurch gekennzeichnet, daß man die in Verfahrensstufe a erhaltene Verbindung der Formel IV durch Säulenchromatographie, vorzugsweise an Kieselgel, in die 15S- und die 15R-Formen auftrennt und in Verfahrensstufe b dann nur die 15S-Form der Verbindung der Formel IV einsetzt.

27. Verfahren zur Herstellung von heterocyclischen Verbindungen gemäß Anspruch 20, dadurch gekennzeichnet, daß in den Verbindungen der Formel I, in denen $R^1$ für ein Kation steht, dieses gegen ein anderes pharmazeutisch verträgliches Kation mit Hilfe der Ionenaustauscherchromatographie ausgetauscht wird.

**Ansprüche** (für den Vertragsstaat AT)

1. Verfahren zur Herstellung von heterocyclischen Verbindungen der Formel

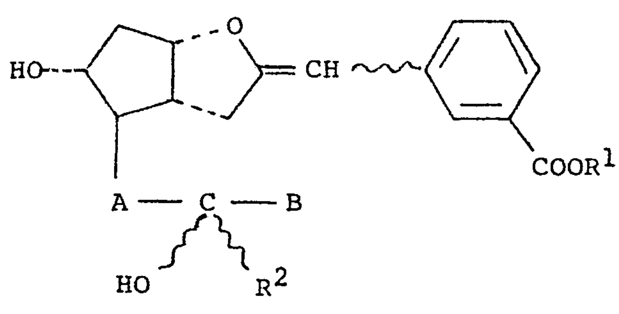

(I)

worin der Phenylrest in Bezug auf die Doppelbindung E- oder Z-Konfiguration aufweisen und am (unter anderem den Rest $R^2$ tragenden) Kohlenstoffatom 15 RS- oder S-Konfiguration vorliegen kann, und worin $R^1$ ein Wasserstoffatom, ein pharmazeutisch verträgliches Kation oder einen geradkettigen oder verzweigten Alkylrest mit 1 bis 6 Kohlenstoffatomen bedeutet, $R^2$ für Wasserstoff oder Methyl steht, A eine der Gruppen $-CH_2-CH_2-$, (trans)-CH = CH— oder —C ≡ C ist und B einen Alkylrest mit 5 bis 9 Kohlenstoffatomen der Struktur

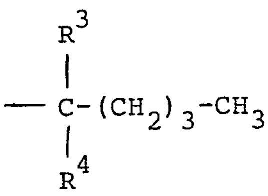

in der $R^3$ und $R^4$ gleich oder verschieden sind und für Wasserstoff, Methyl oder Äthyl stehen, oder einen Cyclohexylrest, der in der 4'-Stellung durch einen Methyl- oder Äthylrest substituiert sein kann, bedeutet, dadurch gekennzeichnet, daß man

a) eine Verbindung der Formel

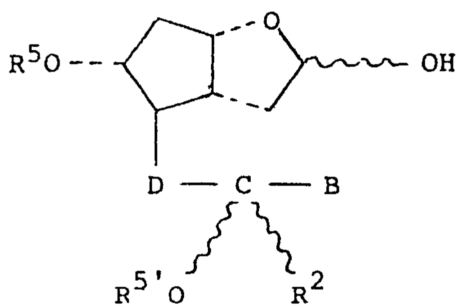

(II)

in der B und $R^2$ die gleiche Bedeutung wie in Formel I haben, D eine $-CH_2-CH_2-$, trans-CH = CH— oder eine —CH = CHal-Gruppe, in der Hal ein Chlor-, Brom- oder Jodatom ist, darstellt und worin $R^5$ und $R^{5'}$ gleich oder verschieden sind und jeweils für ein Wasserstoffatom oder für eine unter milden Bedingungen abspaltbare Schutzgruppe, insbesondere für eine Tetrahydropyranyl- oder eine Trimethylsilylgruppe stehen mit der 1- bis 6-fachen molaren Menge einer Verbindung der Formel

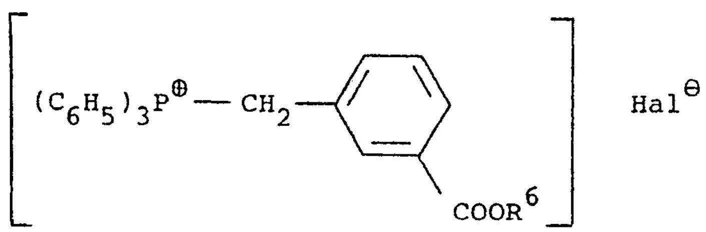

(III)

in der $R^6$ Wasserstoff oder einen Alkylrest mit 1-6 Kohlenstoffatomen, vorzugsweise einen Methyl- oder einen Äthylrest bedeutet und Hal ein Chlor-, Brom- oder Jodatom, vorzugsweise ein Bromatom ist, unter Ausschluß von Sauerstoff bei 10-30 °C in Gegenwart einer starken Base und eines Lösungsmittels umsetzt und gegebenenfalls anschließend, wenn $R^6$ für Wasserstoff steht, die Carboxylgruppe $COOR^6$ vorzugsweise mit Diazomethan bei ca. 0°-30 °C verestert, und, wenn $R^5$ bzw. $R^{5'}$ Schutzgruppen darstellen, diese unter schonenden Bedingungen abspaltet, wobei man eine Verbindung der Formel

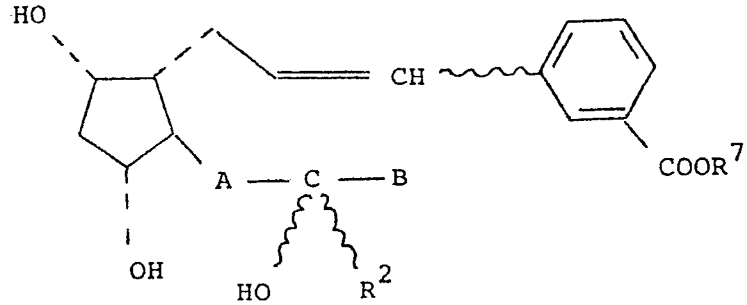

(IV)

in der $R^2$, A und B die gleiche Bedeutung wie in Formel I haben und $R^7$ einen Alkylrest mit 1-6 Kohlenstoffatomen, vorzugsweise einen Methyl- oder einen Äthylrest bedeutet, erhält, diese

b) in Gegenwart eines inerten Lösungsmittels und gegebenenfalls in Gegenwart eines säurebindenden Mittels bei etwa 0°-30 °C bromiert oder jodiert und zu einer Verbindung der Formel

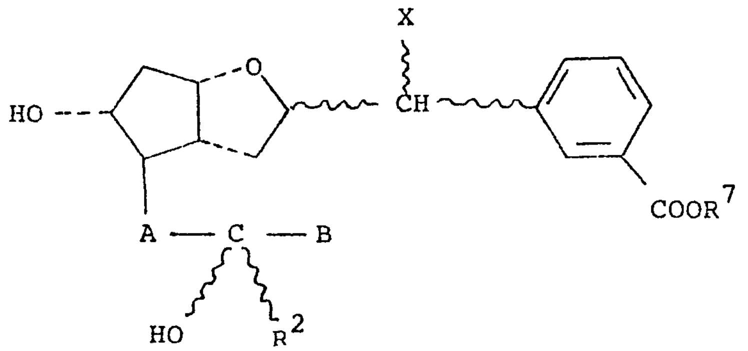

(V)

in der $R^2$, $R^7$, A und B die gleiche Bedeutung wie oben haben und X für Brom oder Jod steht cyclisiert und

c) aus der Verbindung der Formel V bei Temperaturen von etwa 0 bis 90 °C mit der 1- bis 5-fachen molaren Menge einer Base, gegebenenfalls in Anwesenheit eines Lösungsmittels, Halogenwasserstoff HX abspaltet, und gegebenenfalls in der so erhaltenen Verbindung der Formel

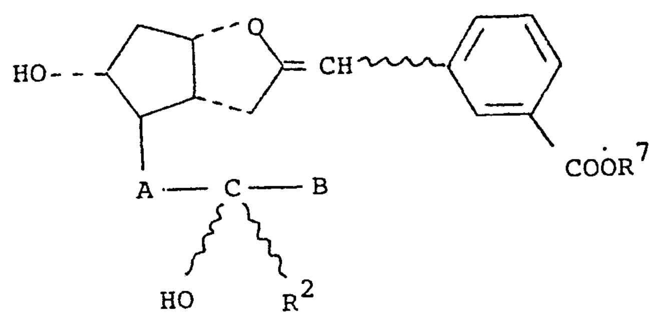

(Ia)

in der $R^2$, $R^7$, A und B die gleiche Bedeutung wie oben haben die Estergruppierung $COOR^7$ in wässrig-alkoholischer Lösung bei etwa 10-50 °C mit einem Alkalihydroxid verseift und vorzugsweise anschließend die so erhaltenen Verbindungen der Formel I in die 5E- und 5Z-Isomeren auftrennt.

2. Verfahren zur Herstellung von heterocyclischen Verbindungen gemäß Anspruch 1, dadurch gekennzeichnet, daß in der Verfahrensstufe a) die Umsetzung der Verbindung der Formel II mit der Verbindung der Formel III unter Zusatz katalytischer Mengen einer Carbonsäure, vorzugsweise von Ameisensäure, Essigsäure, Propionsäure, Bromessigsäure, Trichloressigsäure, Trifluoressigsäure, Phenylessigsäure, p-Nitrobenzoesäure, p-Chlorbenzoesäure, p-Methoxybenzoesäure und insbesondere von Benzoesäure durchgeführt wird.

3. Verfahren zur Herstellung von heterocyclischen Verbindungen gemäß Anspruch 1, dadurch gekennzeichnet, daß in der Verfahrensstufe b zur Halogenierung und Cyclisierung der Verbindung der Formel IV Jod, Jod-Jodkalium oder Bromsuccinimid, gegebenenfalls in Anwesenheit von Wasser und Natriumhydrogencarbonat, Natriumcarbonat, Magnesiumoxid oder Calciumcarbonat, eingesetzt wird.

4. Verfahren zur Herstellung von heterocyclischen Verbindungen gemäß Anspruch 1, dadurch gekennzeichnet, daß in der Verfahrensstufe c die Abspaltung des Halogenwasserstoffes HX mit Hilfe von Natriumhydroxid, Kaliumhydroxid, Natriummethylat, Natriumäthylat, Kalium-tert.-butylat, Triäthylamin, Dicyclohexylamin, 1,5-Diazabicyclo-(4.3.0)-non-5-en oder 1,5-Diazabicyclo-(5.4.0)-undec-5-en erfolgt.

5. Verfahren zur Herstellung von heterocyclischen Verbindungen gemäß Ansprüchen 1 und 4, dadurch gekennzeichnet, daß in der Verfahrensstufe c die Abspaltung des Halogenwasserstoffes HX und die Verseifung der Estergruppe $COOR^7$ ohne intermediäre Isolierung der Verbindung der Formel Ia erfolgt.

6. Verfahren zur Herstellung von heterocyclischen Verbindungen der allgemeinen Formel I in Form der 15S-Isomeren gemäß Anspruch 1, dadurch gekennzeichnet, daß man die RS-Formen der Verbindung der Formel II, IV, V oder Ia bzw. I in an sich bekannter Weise, insbesondere durch Säulenchromatographie, auftrennt.

7. Verfahren gemäß Ansprüchen 1 und 6, dadurch gekennzeichnet, daß man die in Verfahrensstufe a erhaltene Verbindung der Formel IV durch Säulenchromatographie, vorzugsweise an Kieselgel, in die

15S- und die 15R-Formen auftrennt und in Verfahrensstufe b dann nur die 15S-Form der Verbindung der Formel IV einsetzt.

8. Verfahren zur Herstellung von heterocyclischen Verbindungen gemäß Anspruch 1, dadurch gekennzeichnet, daß in den Verbindungen der Formel I, in denen $R^1$ für ein Kation steht, dieses gegen ein anderes pharmazeutisch verträgliches Kation mit Hilfe der Ionenaustauscherchromatographie ausgetauscht wird.

**Claims** (for the Contracting States : BE, CH, DE, FR, GB, IT, LI, LU, NL, SE)

1. Heterocyclic compounds corresponding to the following formula

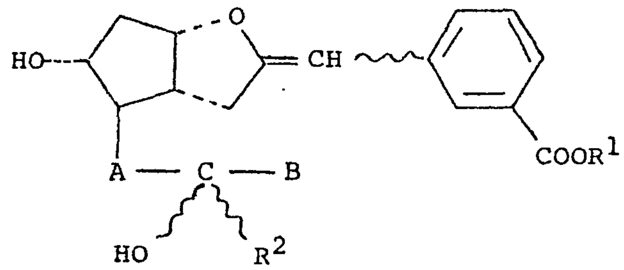

(I)

in which the phenyl radical may have the E- and/or Z-configuration in regard to the double bond whilst the RS- or S-configuration may be present on the carbon atom 15 (carrying *inter alia* the radical $R^2$) and in which $R^1$ represents a hydrogen atom, a pharmaceutically compatible cation or a linear or branched $C_1$-$C_6$ alkyl radical, $R^2$ represents hydrogen or methyl, A represents one of the groups —$CH_2$—$CH_2$—, (trans)-CH = CH— or —C = C— and B represents a $C_5$-$C_9$ alkyl radical having the following structure

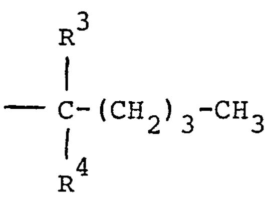

in which $R^3$ and $R^4$ may be the same or different and represent hydrogen, methyl or ethyl, or represents a cyclohexyl radical which may be substituted in the 4'-position by a methyl or ethyl radical.

2. Heterocyclic compounds as claimed in Claim 1 corresponding to the following formula

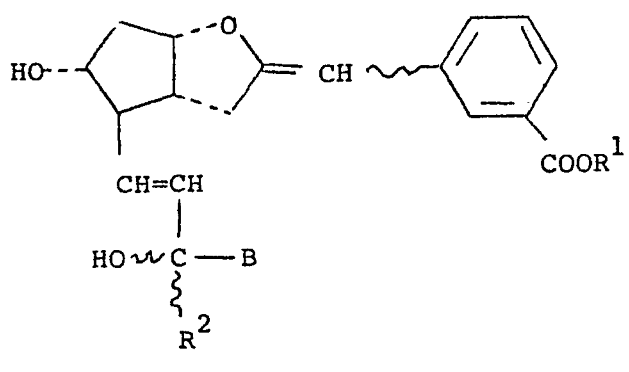

in which $R^1$, $R^2$ and B have the same meaning as in formula I.

3. Heterocyclic compounds as claimed in Claim 1 corresponding to the following formula

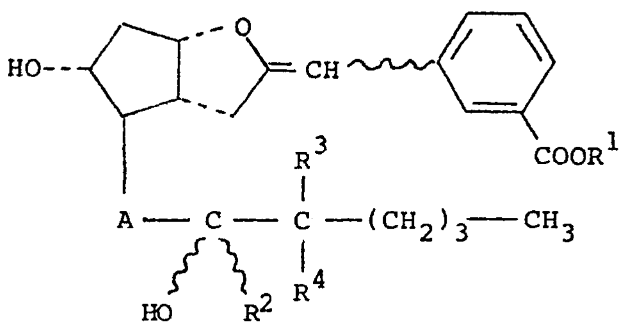

in which $R^1$ to $R^4$ and A have the same meaning as in formula I.

4. Heterocyclic compounds as claimed in Claim 1 corresponding to the following formula

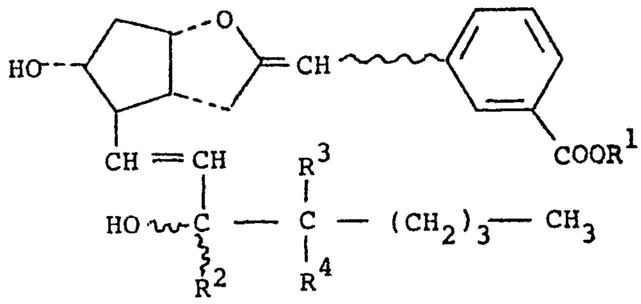

in which $R^1$ to $R^4$ have the same meaning as in formula I.

5. Heterocyclic compounds as claimed in Claim 1 to 4, in which $R^3$ and $R^4$ represent hydrogen.

6. Heterocyclic compounds as claimed in Claims 1 to 4, in which $R^3$ represents hydrogen and $R^4$ represents an ethyl radical.

7. Heterocyclic compounds as claimed in Claim 1 corresponding to the following formula

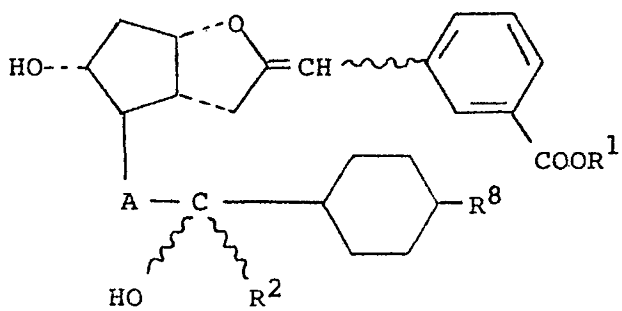

in which $R^1$, $R^2$ and A have the same meaning as in formula I and in which $R^8$ is hydrogen, methyl or ethyl.

8. Heterocyclic compounds as claimed in Claim 1 to 7, in which $R^2$ represents hydrogen.

9. Heterocyclic compounds as claimed in Claim 1 corresponding to the following formula

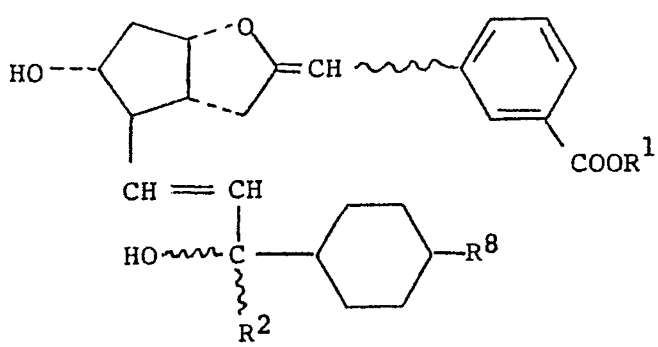

in which $R^1$ and $R^2$ have the same meaning as in formula I and in which $R^8$ represents hydrogen, methyl or ethyl.

10. Heterocyclic compounds as claimed in Claim 1 corresponding to the following formula

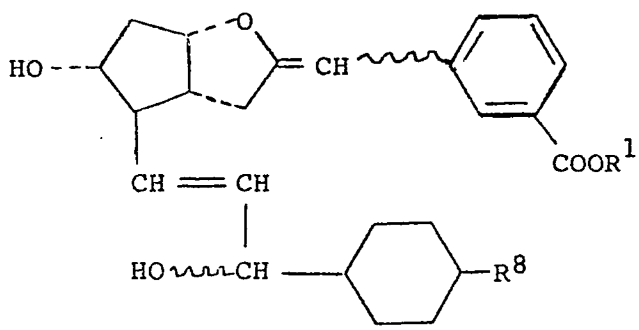

in which $R^1$ has the same meaning as in formula I and in which $R^8$ represents hydrogen, methyl or ethyl.

11. Heterocyclic compounds as claimed in Claims 1 to 10, in which $R^1$ represents a methyl or ethyl radical.

12. Heterocyclic compounds as claimed in claims 1 to 10, in which $R^1$ represents a sodium or potassium ion.

13. The 15 S-forms of the heterocyclic compounds claimed in Claims 1 to 12.

14. The heterocyclic compound [(5EZ, 13E, 9α, 11α, 15S)-2,3,4-trinor-1,5-inter-m-phenylene-6,9-epoxy-11,15-dihydroxy-15-methyl]-prosta-5,13-dienic acid claimed in Claim 1 and its pharmaceutically compatible salts, particularly its sodium salt, and its methyl ester.

15. The heterocyclic compound [(5EZ, 13E, 9α, 11α, 15S)-2,3,4-trinor-1,5-inter-m-phenylene-6,9-epoxy-11,15-dihydroxy-15-cyclohexyl-16,17,18,19,20-pentanor]-prosta-5,13-dienic acid claimed in Claim 1 and its pharmaceutically compatible salts, particularly its sodium salt, and its methyl ester.

16. A medicament, characterized in that it contains as active principle a heterocyclic compound of the type claimed in Claims 1 to 15.

17. A medicament as claimed in Claim 16, characterized in that it contains as active principle from 0.01 to 10 mg of a heterocyclic compound of the type claimed in Claims 1 to 15.

18. A medicament as claimed in Claims 16 and 17, characterized in that it is suitable for parenteral application and contains in a standard liquid sterile vehicle from 0.01 to 1 mg of a heterocyclic compound of the type claimed in Claims 1 to 15 in dissolved or suspended form.

19. A medicament as claimed in Claims 16 and 17, characterized in that it is in the form of tablets, dragees or capsules for oral application and contains from 0.1 to 10 mg of a heterocyclic compound of the type claimed in any of Claims 1 to 15 and standard excipients.

20. A process for producing heterocyclic compounds corresponding to the following formula

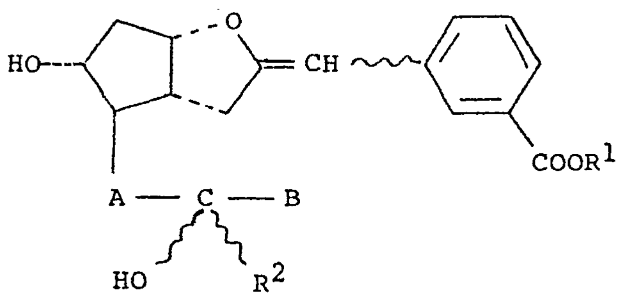

(I)

in which the phenyl radical may have the E- and/or Z-configuration in regard to the double bond whilst the RS- or S-configuration may be present on the carbon atom 15 (carrying *inter alia* the radical $R^2$) and in which $R^1$ represents a hydrogen atom, a pharmaceutically compatible cation or a straight-chain or branched $C_1$-$C_6$-alkyl radical, $R^2$ represents hydrogen or methyl, A represents one of the groups $-CH_2-CH_2-$, (trans)-CH = CH— or $-C \equiv C-$ and B is a $C_5$-$C_9$-alkyl radical having the following structure

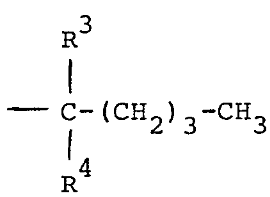

in which $R^3$ and $R^4$ may be the same or different and represent hydrogen, methyl or ethyl, or is a cyclohexyl radical which may be substituted in the 4'-position by a methyl or ethyl radical, characterized in that

a) a compound corresponding to the following formula

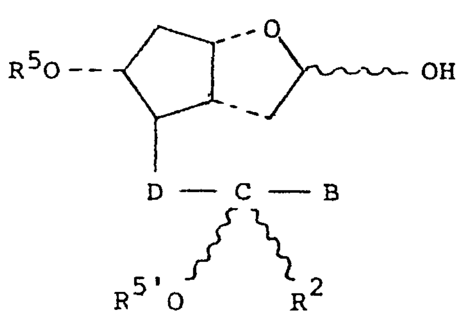

(II)

in which B and $R^2$ have the same meaning as in formula I, D is a $—CH_2—CH_2—$, trans-CH = CH— or —CH = CHal-group, where Hal is a chlorine, bromine or iodine atom, and in which $R^5$ and $R^{5'}$ may be the same or different and each represent a hydrogen atom or a protective group removable under mild conditions, more particularly a tetrahydropyranyl or trimethyl silyl group, is reacted with from 1 to 6 times the molar quantity of a compound corresponding to the following formula

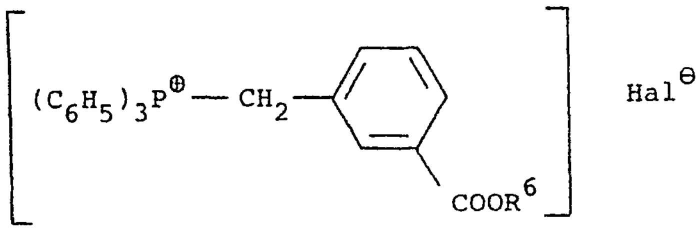

(III)

in which $R^6$ represents hydrogen or a $C_1$-$C_6$-alkyl radical, preferably a methyl or ethyl radical, and Hal is a chlorine, bromine or iodine atom, preferably a bromine atom, in the absence of oxygen at 10 to 30 °C in the presence of a strong base and a solvent and, where $R^6$ represents hydrogen, the carboxyl group $COOR^6$ is then optionally esterified with diazomethane in ether at approximately 0 to 30 °C and, where $R^5$ and $R^{5'}$ are protective groups, these protective groups are split off under mild conditions, resulting in the formation of a compound corresponding to the following formula

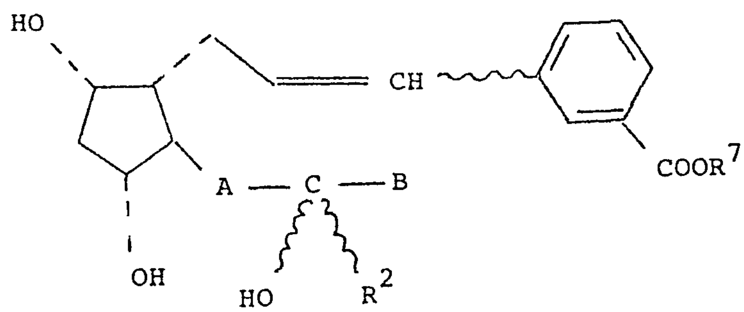

(IV)

in which $R^2$, A and B have the same meaning as in formula I and $R^7$ represents a $C_1$-$C_6$-alkyl radical, preferably a methyl or ethyl radical, the compound thus obtained

b) is halogenated at about 0 to 30 °C in the presence of an inert solvent and optionally in the presence of an acid-binding agent and cyclized to form a compound corresponding to the following formula

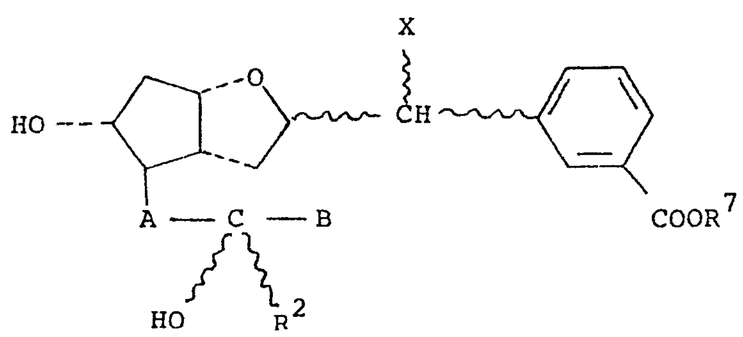

(V)

in which $R^2$, $R^7$, A and B have the same meaning as above and X represents bromine or iodine and

c) hydrogen halide HX is split off from the compound of formula V at temperatures of from about 0 to 90 °C with from 1 to 5 times the molar quantity of a base, optionally in the presence of a solvent, and the ester group $COOR^7$ in the resulting compound which corresponds to the following formula

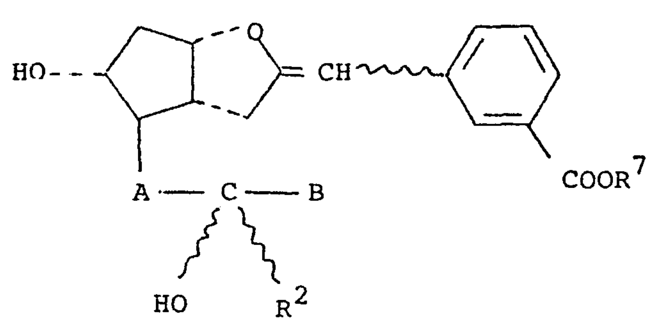

(Ia)

in which $R^2$, $R^7$, A and B have the same meaning as above, is optionally hydrolyzed with an alkali hydroxide at around 10 to 50 °C in aqueous-alcoholic solution and the resulting compounds of formula I are then preferably separated up into the 5E- and 5Z-isomers.

21. A process for the production of heterocyclic compounds as claimed in claim 20, characterized in that, in step a), the reaction of the compound corresponding to formula II with the compound corresponding to formula III is carried out in the presence of catalytic quantities of a carboxylic acid, preferably formic acid, acetic acid, propionic acid, bromoacetic acid, trichloroacetic acid, trifluoroacetic acid, phenyl acetic acid, p-nitrobenzoic acid, p-chlorobenzoic acid, p-methoxy benzoic acid and, more particularly, benzoic acid.

22. A process for the production of heterocyclic compounds as claimed in Claim 20, characterized in that, in step b), iodine, iodine-potassium iodide or bromosuccinimide, optionally in the presence of water and sodium hydrogen carbonate, sodium carbonate, magnesium oxide or calcium carbonate, is used for halogenating and cyclizing the compound corresponding to formula IV.

23. A process for the production of heterocyclic compounds as claimed in Claim 20, characterized in that, in step c) the hydrogen halide HX is split off by means of sodium hydroxide, potassium hydroxide, sodium methylate, sodium ethylate, potassium-tert.-butylate, triethylamine, dicyclohexylamine, 1,5-diazabicyclo-(4.3.0)-non-5-ene or 1,5-diazabicyclo-(5.4.0)-undec-5-ene.

24. A process for the production of heterocyclic compounds as claimed in Claims 20 and 23, characterized in that, in step c), the hydrogen halide HX is split off and the ester group $COOR^7$ hydrolyzed without intermediate isolation of the compound corresponding to formula Ia.

25. A process for the production of heterocyclic compounds corresponding to general formula I in the form of the 15S-isomers as claimed in Claim 20, characterized in that the RS-forms of the compound of formula II, IV, V or Ia or I are separated up in known manner, more particularly by column chromatography.

26. A process as claimed in Claim 20 and 25, characterized in that the compound of formula IV obtained in step a) is separated up into the 15S- and the 15R-forms by column chromatography, preferably using silica gel, and thereafter only the 15S-form of the compound of formula IV is used in step b).

27. A process for the production of heterocyclic compounds as claimed in Claim 20, characterized in that in the compounds of formula I, in which $R^1$ represents a cation, this cation is replaced by another pharmaceutically compatible cation by ion exchange chromatography.

**Claims** (for the Contracting State AT)

1. A process for producing heterocyclic compounds corresponding to the following formula

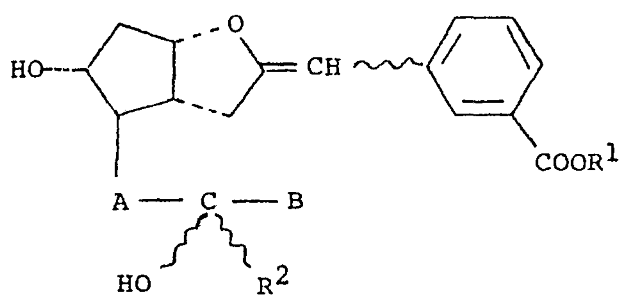

(I)

in which the phenyl radical may have the E- and/or Z-configuration in regard to the double bond whilst the RS- or S-configuration may be present on the carbon atom 15 (carrying *inter alia* the radical $R^2$) and in which $R^1$ represents a hydrogen atom, a pharmaceutically compatible cation or a straight-chain or branched $C_1$-$C_6$-alkyl radical, $R^2$ represents hydrogen or methyl, A represents one of the groups $-CH_2-CH_2-$, (trans)-$CH=CH-$ or $-C\equiv C-$ and B is a $C_5$-$C_9$-alkyl radical having the following structure

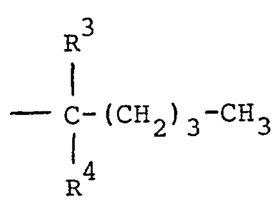

in which $R^3$ and $R^4$ may be the same or different and represent hydrogen, methyl or ethyl, or is a

cyclohexyl radical which may be substituted in the 4'-position by a methyl or ethyl radical, characterized in that

a) a compound corresponding to the following formula

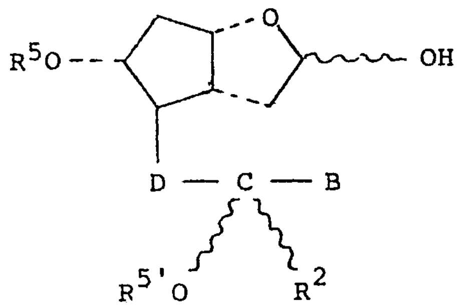

(II)

in which B and $R^2$ have the same meaning as in formula I, D is a $-CH_2-CH_2-$, trans-CH = CH— or —CH = CHal-group, where Hal is a chorine, bromine or iodine atom, and in which $R^5$ and $R^{5'}$ may be the same or different and each represent a hydrogen atom or a protective group removable under mild conditions, more particularly a tetrahydropyranyl or trimethyl silyl group, is reacted with from 1 to 6 times the molar quantity of a compound corresponding to the following formula

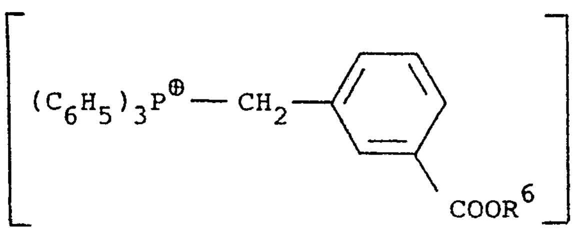

(III)

in which $R^6$ represents hydrogen or a $C_1$-$C_6$-alkyl radical, preferably a methyl or ethyl radical, and Hal is a chlorine, bromine or iodine atom, preferably a bromine atom, in the absence of oxygen at 10 to 30 °C in the presence of a strong base and a solvent and, where $R^6$ represents hydrogen, the carboxyl group $COOR^6$ is then optionally esterified with diazomethane at approximately 0 to 30 °C and, where $R^5$ and $R^{5'}$ are protective groups, these protective groups are split off under mild conditions, resulting in the formation of a compound corresponding to the following formula

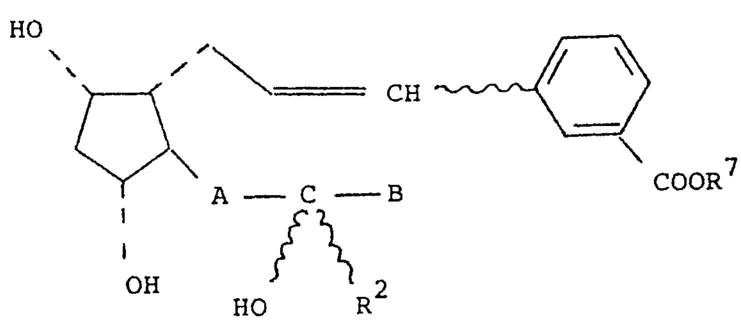

(IV)

in which $R^2$, A and B have the same meaning as in formula I and $R^7$ represents a $C_1$-$C_6$-alkyl radical, preferably a methyl or ethyl radical, the compound thus obtained

b) is brominated or iodinated at about 0 to 30 °C in the presence of an inert solvent and optionally in the presence of an acid-binding agent and cyclized to form a compound corresponding to the following formula

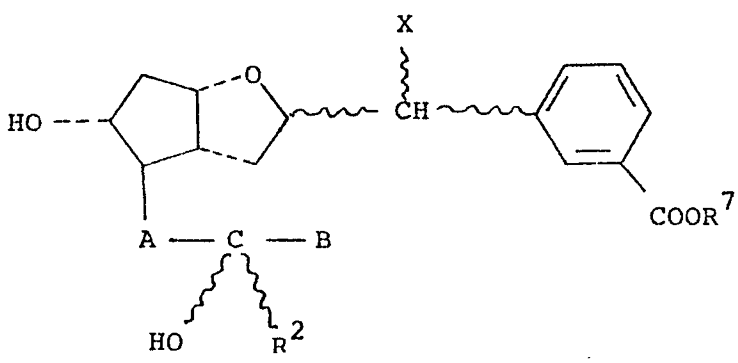

(V)

in which $R^2$, $R^7$, A and B have the same meaning as above and X represents bromine or iodine and

c) hydrogen halide HX is split off from the compound of formula V at temperatures of from about 0 to 90 °C with from 1 to 5 times the molar quantity of a base, optionally in the presence of a solvent, and the ester group $COOR^7$ in the resulting compound which corresponds to the following formula

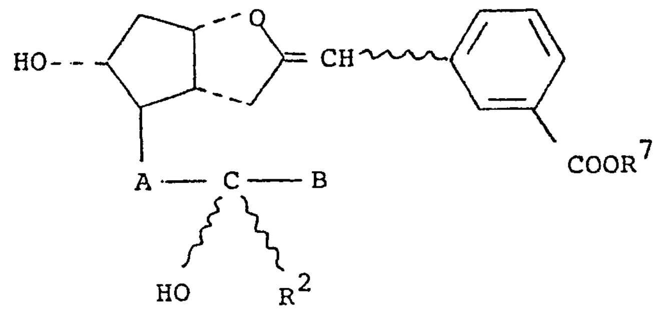

(Ia)

in which $R^2$, $R^7$, A and B have the same meaning as above, is optionally hydrolyzed with an alkali hydroxide at around 10 to 50 °C in aqueous-alcoholic solution and the resulting compounds of formula I are then preferably separated up into the 5E- and 5Z-isomers.

2. A process for the production of heterocyclic compounds as claimed in claim 1, characterized in that, in step a), the reaction of the compound corresponding to formula II with the compound corresponding to formula III is carried out in the presence of catalytic quantities of a carboxylic acid, preferably formic acid, acetic acid, propionic acid, bromoacetic acid, trichloroacetic acid, trifluoroacetic acid, phenyl acetic acid, p-nitrobenzoic acid, p-chlorobenzoic acid, p-methoxy benzoic acid and, more particularly, benzoic acid.

3. A process for the production of heterocyclic compounds as claimed in Claim 1, characterized in that, in step b), iodine, iodine-potassium iodide or bromosuccinimide, optionally in the presence of water and sodium hydrogen carbonate, sodium carbonate, magnesium oxide or calcium carbonate, is used for halogenating and cyclizing the compound corresponding to formula IV.

4. A process for the production of heterocyclic compounds as claimed in Claim 1, characterized in that, in step c) the hydrogen halide HX is split off by means of sodium hydroxide, potassium hydroxide, sodium methylate, sodium ethylate, potassium-tert.-butylate, triethylamine, dicyclohexylamine, 1,5-diazabicyclo-(4.3.0)-non-5-ene or 1,5-diazabicyclo-(5.4.0)-undec-5-ene.

5. A process for the production of heterocyclic compounds as claimed in Claims 1 and 4, characterized in that, in step c), the hydrogen halide HX is split off and the ester group $COOR^7$ hydrolyzed without intermediate isolation of the compound corresponding to formula Ia.

6. A process for the production of heterocyclic compounds corresponding to general formula I in the form of the 15S-isomers as claimed in Claim 1, characterized in that the RS-forms of the compound of formula II, IV, V or Ia or I are separated up in known manner, more particularly by column chromatography.

7. A process as claimed in Claim 1 and 6, characterized in that the compound of formula IV obtained in step a) is separated up into the 15S- and the 15R-forms by column chromatography, preferably using silica gel, and thereafter only the 15S-form of the compound of formula IV is used in step b).

8. A process for the production of heterocyclic compounds as claimed in Claim 1, characterized in that in the compounds of formula I, in which $R^1$ represents a cation, this cation is replaced by another pharmaceutically compatible cation by ion exchange chromatography.

**Revendications** (pour les Etats contractants : BE, CH, DE, FR, GB, IT, LI, LU, NL, SE)

1. Composés hétérocycliques de formule

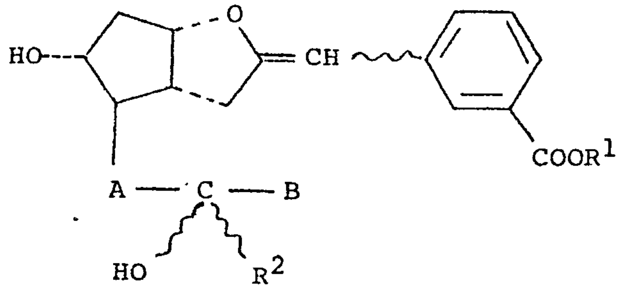

(I)

dans laquelle le reste phényle peut présenter par rapport à la double liaison la configuration E et/ou Z et la configuration RS ou S peut exister sur l'atome de carbone 15 (portant entre autres le reste $R^2$) et $R^1$ est une atome d'hydrogène, un cation acceptable du point de vue pharmaceutique ou un reste alkyle à chaîne droite ou ramifié ayant 1 à 6 atomes de carbone, $R^2$ représente l'hydrogène ou le groupe méthyle, A est l'un des groupes $—CH_2—CH_2—$, (trans)-CH = CH— ou $—C \equiv C—$ et B est un reste alkyle ayant 5 à 9 atomes de carbone, de formule

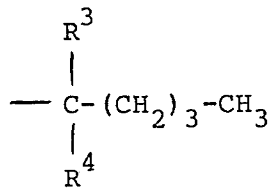

dans laquelle $R^3$ et $R^4$ sont égaux ou différents et désignent l'hydrogène, le groupe méthyle ou le groupe éthyle, ou un reste cyclohexyle qui peut être substitué en position 4' par un reste méthyle ou éthyle.

2. Composés hétérocycliques suivant la revendication 1, répondant à la formule

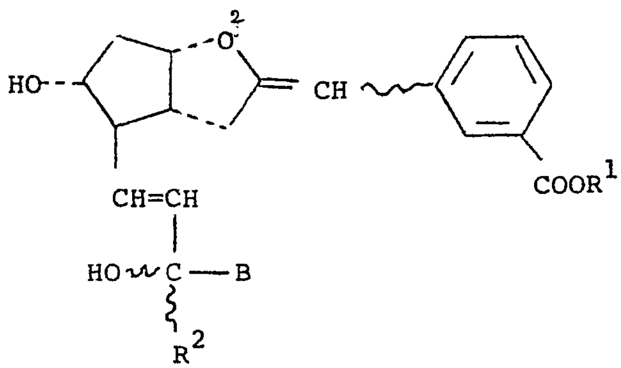

dans laquelle $R^1$, $R^2$ et B ont la même définition que pour la formule I.

3. Composés hétérocycliques suivant la revendication 1, correspondant à la formule

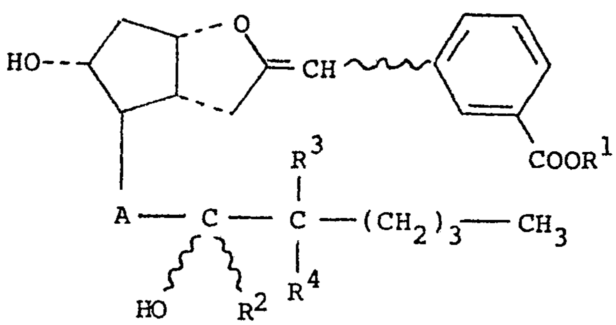

dans laquelle $R^1$ à $R^4$ et A ont la même définition que pour la formule I.

4. Composés hétérocycliques suivant la revendication 1, correspondant à la formule

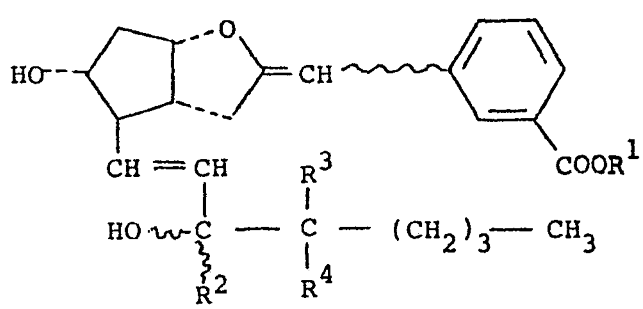

dans laquelle $R^1$ à $R^4$ ont la même définition que pour la formule I.

5. Composés hétérocycliques suivant les revendications 1-4, dans lesquels $R^3$ et $R^4$ représentent de l'hydrogène.

6. Composés hétérocycliques suivant les revendications 1-4, dans lesquels $R^3$ est l'hydrogène et $R^4$ est un reste éthyle.

7. Composés hétérocycliques suivant la revendication 1, correspondant à la formule

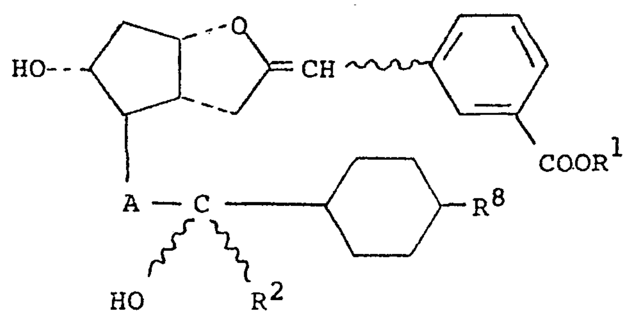

dans laquelle $R^1$, $R^2$ et A ont la même définition que pour la formule I et $R^8$ représente l'hydrogène, le groupe méthyle, ou le groupe éthyle.

8. Composés hétérocycliques suivant les revendications 1-7, dans lesquels $R^2$ représente l'hydrogène.

9. Composés hétérocycliques suivant la revendication 1, correspondant à la formule

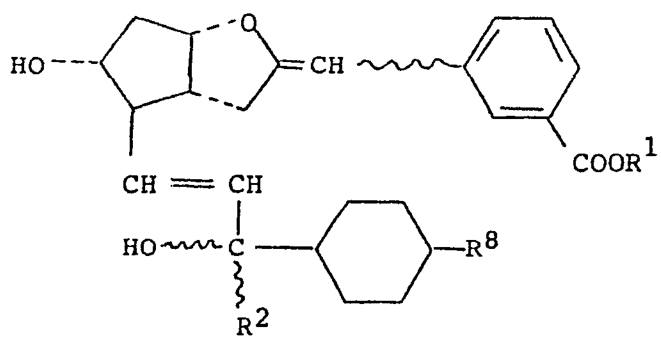

dans laquelle $R^1$ et $R^2$ ont la même définition que pour la formule I et $R^8$ représente l'hydrogène, le groupe méthyle ou le groupe éthyle.

10. Composés hétérocycliques suivant la revendication 1, correspondant à la formule

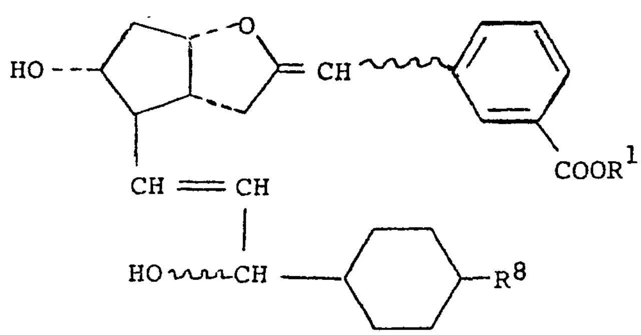

dans laquelle $R^1$ a la même définition que pour la formule I et $R^8$ représente l'hydrogène, le groupe méthyle ou le groupe éthyle.

11. Composés hétérocycliques suivant les revendications 1-10, dans lesquels $R^1$ est un reste méthyle ou un reste éthyle.

12. Composés hétérocycliques suivant les revendications 1-10, dans lesquels $R^1$ est un ion sodium ou un ion potassium.

13. Les formes 15S des composés hétérocycliques suivant les revendications 1 à 12.

14. Le composé hétérocyclique acide [(5EZ, 13E, 9α, 11α, 15S)-2,3,4-trinor-1,5-inter-m-phénylène-6,9-époxy-11,15-dihydroxy-15-méthyl]-prosta-5,13-diénique et ses sels pharmaceutiquement acceptables suivant la revendication 1, notamment son sel de sodium et son ester méthylique.

15. Le composé hétérocyclique acide [(5EZ, 13E, 9α, 11α, 15S)-2,3,4-trinor-1,5-inter-m-phénylène-6,9-

époxy-11,15-dihydroxy-15-cyclohexyl-16,17,18,19,20-pentanor]-prosta-5,13-diénique et ses sels pharmaceutiquement acceptables suivant la revendication 1, notamment son sel de sodium et son ester méthylique.

16. Médicament, caractérisé en ce qu'il contient comme substance active un composé hétérocyclique correspondant aux revendications 1-15.

17. Médicament suivant la revendication 16, caractérisé en ce qu'il contient comme substance active 0,01 à 10 mg d'un composé hétérocyclique correspondant aux revendications 1-15.

18. Médicament suivant les revendications 16 et 17, caractérisé en ce qu'il convient à l'administration parentérale et en ce qu'il contient dans un support stérile liquide classique 0,01 à 1 mg d'un composé hétérocyclique suivant les revendications 1-15 sous la forme dissoute ou en suspension.

19. Médicament suivant les revendications 16 et 17, caractérisé en ce qu'il se présente sous la forme de comprimés, dragées ou capsules pour l'administration orale et contient 0,1 à 10 mg d'un composé hétérocyclique correspondant aux revendications 1-15 ainsi que des supports classiques.

20. Procédé de production de composés hétérocycliques de formule

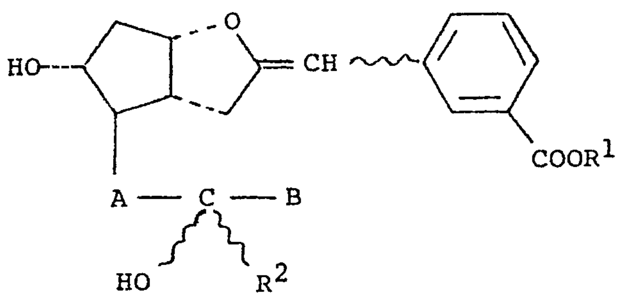

(I)

dans laquelle le reste phényle peut présenter par rapport à la double liaison la configuration E et/ou Z et la configuration RS ou S peut exister sur l'atome de carbone 15 (portant entre autres le reste $R^2$) et $R^1$ est un atome d'hydrogène, un cation acceptable du point de vue pharmaceutique ou un reste alkyle à chaîne droite ou ramifié ayant 1 à 6 atomes de carbone, $R^2$ représente l'hydrogène ou le groupe méthyle, A est l'un des groupes $—CH_2—CH_2—$, (trans)-CH = CH— ou —C ≡ C— et B est un reste alkyle ayant 5 à 9 atomes de carbone, de formule

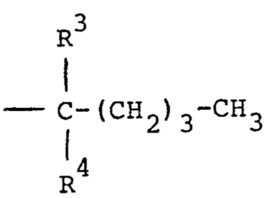

dans laquelle $R^3$ et $R^4$ sont égaux ou différents et désignent l'hydrogène, le groupe méthyle ou le groupe éthyle, ou un reste cyclohexyle qui peut être substitué en position 4' par un reste méthyle ou éthyle, caractérisé en ce que :

a) on fait réagir un composé de formule

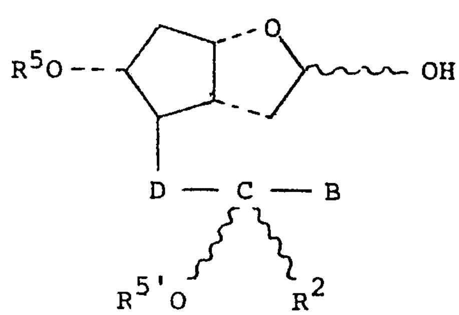

(II)

dans laquelle B et $R^2$ ont la même définition que dans la formule I, D est un groupe $—CH_2—CH_2—$, trans-CH = CH— ou un groupe —CH = CHal—, dans lequel Hal est un atome de chlore, de brome ou d'iode, et $R^5$ et $R^{5'}$ sont égaux ou différents et représentent chacun un atome d'hydrogène ou un groupe protecteur pouvant être éliminé dans des conditions douces, notamment un groupe tétrahydropyrannyle ou un groupe triméthylsilyle avec 1 à 6 fois la quantité molaire d'un composé de formule

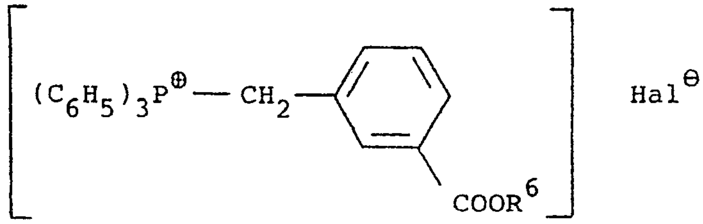

(III)

dans laquelle $R^6$ représente l'hydrogène ou un reste alkyle ayant 1 à 6 atomes de carbone, de préférence un reste méthyle ou un reste éthyle et Hal désigne un atome de chlore, de brome ou d'iode, de préférence un atome de brome, à l'abri de l'oxygène à 10-30 °C en présence d'une base forte et d'un solvant puis, le cas échéant, lorsque $R^6$ représente l'hydrogène, on estérifie le groupe carboxyle $COOR^6$ avec le diazométhane dans l'éther à environ 0-30 °C et, lorsque $R^5$ ou $R^{5'}$ représente des groupes protecteurs, on élimine ces groupes dans des conditions douces et on obtient ainsi un composé de formule

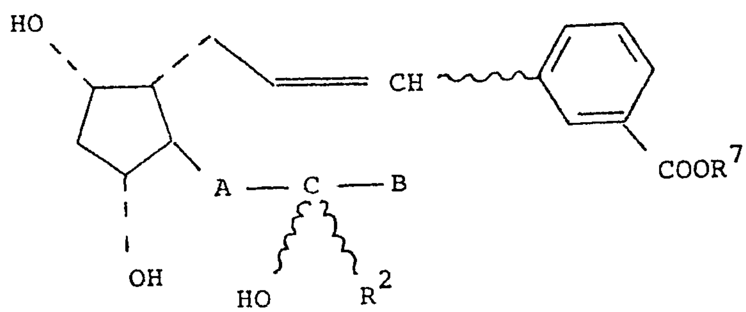

(IV)

dans laquelle $R^2$, A et B ont la même définition que dans la formule I et $R^7$ est un reste alkyle ayant 1 à 6 atomes de carbone, de préférence un reste méthyle ou un reste éthyle,

b) on halogène ce composé en présence d'un solvant inerte et le cas échéant en présence d'un accepteur d'acide à environ 0-30 °C et on le cyclise en un composé de formule

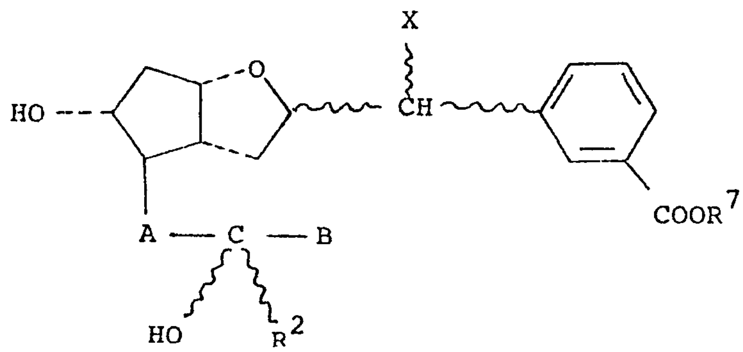

(V)

dans laquelle $R^2$, $R^7$, A et B ont la même définition que ci-dessus et X représente le brome ou l'iode et

c) on élimine l'halogénure d'hydrogène HX du composé de formule V à des températures d'environ 0 à 90 °C avec une à cinq fois la quantité molaire d'une base, éventuellement en présence d'un solvant et, dans le composé ainsi obtenu de formule

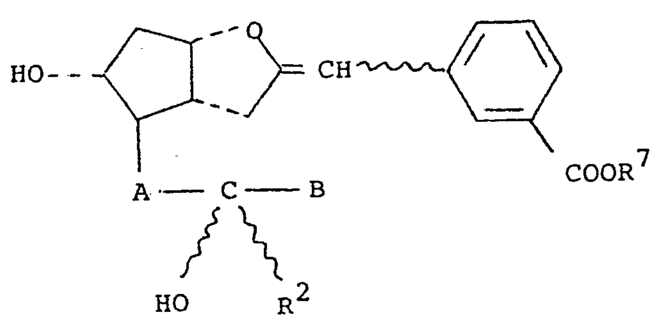

(Ia)

dans laquelle $R^2$, $R^7$, A et B ont la même définition que ci-dessus, on saponifie éventuellement le groupement ester $COOR^7$ en solution hydroalcoolique à environ 10-50 °C avec un hydroxyde alcalin puis on sépare avantageusement les composés de formule I ainsi obtenus en les isomères 5E et 5Z.

21. Procédé de production de composés hétérocycliques suivant la revendication 20, caractérisé en ce que dans l'étape a) du procédé, on conduit la réaction du composé de formule II avec le composé de formule III avec l'addition de quantités catalytiques d'un acide carboxylique, de préférence l'acide formique, l'acide acétique, l'acide propionique, l'acide bromacétique, l'acide trichloracétique, l'acide trifluoracétique, l'acide phénylacétique, l'acide p-nitrobenzoïque, l'acide p-chlorobenzoïque, l'acide p-méthoxybenzoïque et notamment l'acide benzoïque.

22. Procédé de production de composés hétérocycliques suivant la revendication 20, caractérisé en ce que dans l'étape b) du procédé, on utilise pour l'halogénation et la cyclisation du composé de formule IV l'iode, un mélange d'iode et d'iodure de potassium ou le bromosuccinimide, éventuellement en présence d'eau et de bicarbonate de sodium, de carbonate de sodium, d'oxyde de magnésium ou de carbonate de calcium.

23. Procédé de production de composés hétérocycliques suivant la revendication 20, caractérisé en ce que dans l'étape c) du procédé, on effectue l'élimination de l'halogénure d'hydrogène HX à l'aide d'hydroxyde de sodium, d'hydroxyde de potassium, de méthylate de sodium, d'éthylate de sodium, de tertio-butylate de potassium, de triéthylamine, de cyclohexylamine, de 1,5-diazabicyclo-(4.3.0)-nonène-5 ou de 1,5-diazabicyclo-(5.4.0)-undécène-5.

24. Procédé de production de composés hétérocycliques suivant les revendications 20 et 23, caractérisé en ce que dans l'étape c) du procédé, on effectue l'élimination de l'halogénure d'hydrogène HX et la saponification du groupe ester $COOR^7$ sans isolement intermédiaire du composé de formule Ia.

25. Procédé de production de composés hétérocycliques de formule générale I sous la forme des isomères 15S suivant la revendication 20, caractérisé en ce qu'on sépare les formes RS du composé de formule II, IV, V ou Ia ou respectivement I d'une manière connue, notamment par chromatographie sur colonne.

26. Procédé suivant les revendications 20 et 25, caractérisé en ce qu'on sépare le composé de formule IV obtenu dans l'étape a) du procédé par chromatographie sur colonne, de préférence sur gel de silice, en les formes 15S et 15R et on utilise ensuite uniquement la forme 15S du composé de formule IV dans l'étape b) du procédé.

27. Procédé de production de composés hétérocycliques suivant la revendication 20, caractérisé en ce que dans les composés de formule I dans lesquels $R^1$ représente un cation, ce cation est échangé contre un autre cation pharmaceutiquement acceptable à l'aide de la chromatographie par échange ionique.

**Revendications** (pour l'Etat contractant AT)

1. Procédé de production de composés hétérocycliques de formule

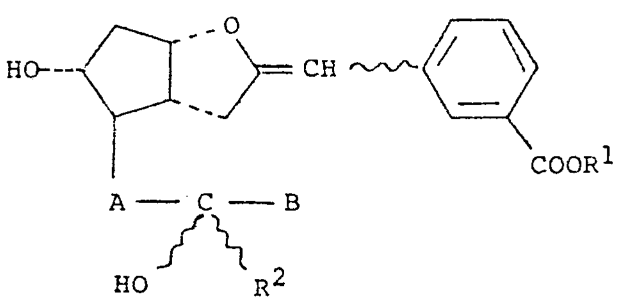

(I)

dans laquelle le reste phényle peut présenter par rapport à la double liaison la configuration E ou Z et la configuration RS ou S peut exister sur l'atome de carbone 15 (portant entre autres le reste $R^2$), et $R^1$ est un atome d'hydrogène, un cation acceptable du point de vue pharmaceutique ou un reste alkyle à chaîne droite ou ramifié ayant 1 à 6 atomes de carbone, $R^2$ représente l'hydrogène ou le groupe méthyle, A est l'un des groupes $—CH_2—CH_2—$, (trans)-CH = CH— ou $—C \equiv C—$ et B est un reste alkyle ayant 5 à 9 atomes de carbone, de formule

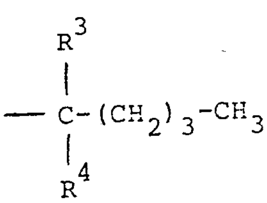

dans laquelle $R^3$ et $R^4$ sont égaux ou différents et désignent l'hydrogène, le groupe méthyle ou le groupe éthyle, ou un reste cyclohexyle qui peut être substitué en position 4' par un reste méthyle ou éthyle, caractérisé en ce que,

a) on fait réagir un composé de formule

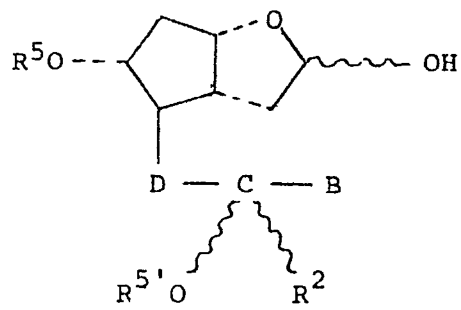

(II)

dans laquelle B et $R^2$ ont la même définition que dans la formule I, D est un groupe $-CH_2-CH_2-$, trans-$CH=CH-$ ou un groupe $-CH=CHal-$, dans lequel Hal est un atome de chlore, de brome ou d'iode, et $R^5$ et $R^{5'}$ sont égaux ou différents et représentent chacun un atome d'hydrogène ou un groupe protecteur pouvant être éliminé dans des conditions douces, notamment un groupe tétrahydropyrannyle ou un groupe tétraméthylsilyle avec la quantité 1 à 6 fois molaire d'un composé de formule

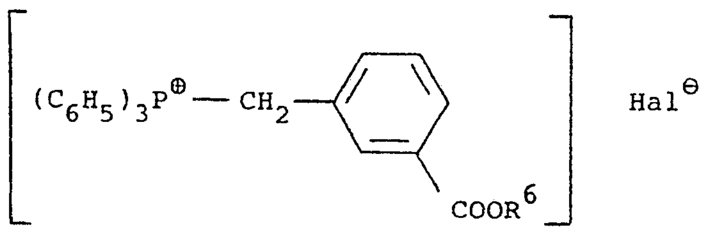

(III)

dans laquelle $R^6$ représente l'hydrogène ou un reste alkyle ayant 1 à 6 atomes de carbone, de préférence un reste méthyle ou un reste éthyle et Hal est un atome de chlore, de brome ou d'iode, de préférence un atome de brome, à l'abri de l'oxygène à 10-30 °C en présence d'une base forte et d'un solvant puis, le cas échéant, lorsque $R^6$ désigne l'hydrogène, on estérifie le groupe carboxyle $COOR^6$ de préférence avec le diazométhane à environ 0-30 °C et, lorsque $R^5$ ou $R^{5'}$ représente des groupes protecteurs, on élimine ces groupes dans des conditions douces, et on obtient alors un composé de formule

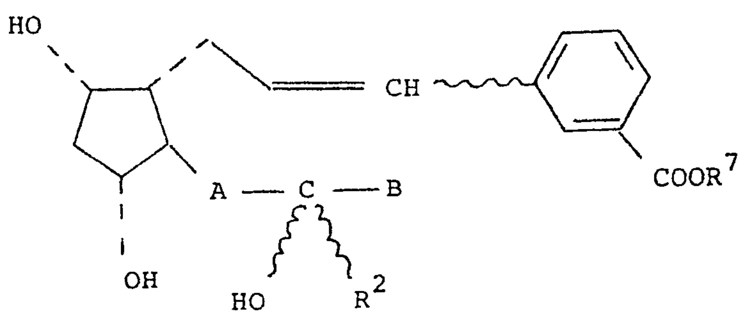

(IV)

dans laquelle $R^2$, A et B ont la même définition que dans la formule I et $R^7$ est un reste alkyle ayant 1 à 6 atomes de carbone, de préférence un reste méthyle ou un reste éthyle,

b) on brome ou on iode ce composé en présence d'un solvant inerte et, le cas échéant, en présence d'un accepteur d'acide à environ 0-30 °C et on les cyclise en un composé de formule

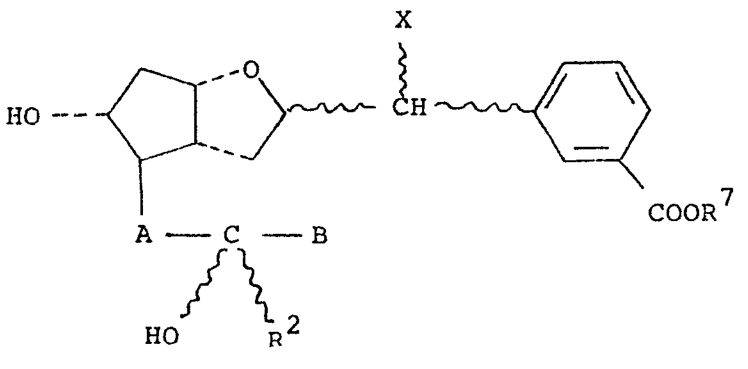

(V)

dans laquelle $R^2$, $R^7$, A et B ont la même définition que ci-dessus et X désigne le brome ou l'iode et

c) on élimine l'halogénure d'hydrogène HX du composé de formule V à des températures d'environ 0 à 90 °C avec la quantité 1 à 5 fois molaire d'une base, éventuellement en présence d'un solvant et, le cas échéant, on saponifie dans le composé ainsi obtenu de formule

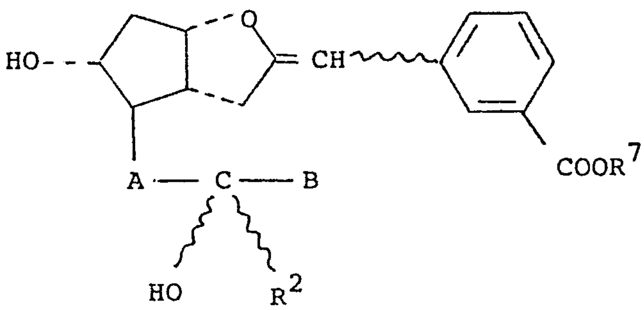

(Ia)

dans laquelle $R^2$, $R^7$, A et B ont la même définition que ci-dessus le groupement ester $COOR^7$ en solution hydro-alcoolique à environ 10-50 °C avec un hydroxyle alcalin puis on divise ensuite les composés de formule I ainsi obtenus en les isomères 5E et 5Z.

2. Procédé de production de composés hétérocycliques suivant la revendication 1, caractérisé en ce que dans l'étape a) du procédé, on conduit la réaction du composé de formule II avec le composé de formule III avec addition de quantités catalytiques d'un acide carboxylique, de préférence l'acide formique, l'acide acétique, l'acide propionique, l'acide bromacétique, l'acide trichloracétique, l'acide trifluoracétique, l'acide phénylacétique, l'acide p-nitrobenzoïque, l'acide p-chlorobenzoïque, l'acide p-méthoxybenzoïque et notamment l'acide benzoïque.

3. Procédé de production de composés hétérocycliques suivant la revendication 1, caractérisé en ce qu'on utilise dans l'étape b) du procédé pour l'halogénation et la cyclisation du composé IV, l'iode, un mélange d'iode et d'iodure de potassium ou le bromosuccinimide, éventuellement en présence d'eau et de bicarbonate de sodium, de carbonate de sodium, d'oxyde de magnésium ou de carbonate de calcium.

4. Procédé de production de composés hétérocycliques suivant la revendication 1, caractérisé en ce que dans l'étape c) du procédé, l'élimination de l'halogénure d'hydrogène HX est effectuée à l'aide d'hydroxyde de sodium, d'hydroxyde de potassium, de méthylate de sodium, d'éthylate de sodium, de tertio-butylate de potassium, de triéthylamine, de dicyclohexylamine, de 1,5-diazabicyclo-(4.3.0)-non-5-ène ou de 1,5-diazabicyclo-(5.4.0)-undéc-5-ène.

5. Procédé de production de composés hétérocycliques suivant les revendications 1 et 4, caractérisé en ce que dans l'étape c) du procédé, l'élimination de l'halogénure d'hydrogène HX et la saponification du groupe ester $COOR^7$ sont effectuées sans isolement intermédiaire du composé de formule Ia.

6. Procédé de production de composés hétérocycliques de formule générale I sous la forme des isomères 15S suivant la revendication 1, caractérisé en ce qu'on sépare les formes RS du composé de formule II, IV, V ou Ia ou, respectivement, I d'une manière connue, notamment par chromatographie sur colonne.

7. Procédé suivant les revendications 1 et 6, caractérisé en ce qu'on divise le composé de formule IV obtenu dans l'étape a) du procédé, par chromatographie sur colonne, de préférence sur gel de silice, en les formes 15S et 15R et on utilise ensuite uniquement la forme 15S du composé de formule IV dans l'étape b) du procédé.

8. Procédé de production de composés hétérocycliques suivant la revendication 1, caractérisé en ce que dans les composés de formule I dans lesquels $R^1$ représente un cation, on échange ce cation contre un autre cation pharmaceutiquement acceptable à l'aide de la chromatographie sur échangeurs d'ions.